# FASCICULE DE BREVET EUROPEEN

(11) **EP 1 232 180 B1**
(45) Date de publication et mention de la délivrance du brevet: **10.01.2007**
(21) Numéro de dépôt: 00981458.3
(22) Date de dépôt: 22.11.2000
(51) Int. Cl.: C07K 14/705, C12N 15/10, C12N 15/12, C12N 15/63, G01N 33/53, A61K 38/17, A61P 33/14, A61P 35/00, A61P 3/14

(54) **POLYPEPTIDES DERIVES DU RECEPTEUR NUCLEAIRE DE LA VITAMINE D, ET LEURS UTILISATIONS NOTAMMENT DANS LE CADRE DU CRIBLAGE D'ANALOGUES DE LA VITAMINE D**
POLYPEPTIDE AUS DEN ZELLKERNREZEPTOR DES VITAMINS D, UND IHRE VERWENDUNG INSBESONDERE ZUM SCREENING VON VITAMIN D-ANALOGA
POLYPEPTIDES DERIVED FROM VITAMIN D NUCLEAR RECEPTOR, AND THEIR USES IN PARTICULAR FOR SCREENING VITAMIN D ANALOGUES

(30) Priorité: 22.11.1999 FR 9914633
(43) Date de publication de la demande: 21.08.2002
(73) Titulaire: CENTRE NATIONAL DE LA RECHERCHE SCIENTIFIQUE (CNRS), 75794 Paris Cedex 16 (FR)
(72) Inventeur: MORAS, Dino, F-67450 Lampertheim (FR); ROCHEL , épouse GUIBERTEAU , Natacha, F-67300 Schiltigheim (FR); WURTZ, Jean-Marie, F-67410 Drusenheim (FR)
(74) Mandataire: Grosset-Fournier, Chantal Catherine
(86) Numéro de dépôt international: PCT/FR2000/003248
(87) Numéro de publication internationale: WO 2001/038393

(56) Documents cités:
- WO-A-98/31835
- WO-A-99/16872
- WO-A-99/19354
- WO-A-99/50658
- WURTZ J -M ET AL: "A CANONICAL STRUCTURE FOR THE LIGAND-BINDING DOMAIN OF NUCLEAR RECEPTORS" NATURAL STRUCTURAL BIOLOGY, vol. 3, 1996, pages 87-94, XP000610199 cité dans la demande
- RENAUD JEAN-PAUL ET AL: "Crystal structure of the RAR-gamma ligand-binding domain bound to all-trans retinoic acid." NATURE (LONDON), vol. 378, no. 6558, 1995, pages 681-689, XP000885365 ISSN: 0028-0836 cité dans la demande
- BAKER A R ET AL: "CLONING AND EXPRESSION OF FULL-LENGTH COMPLEMENTARY DNA ENCODING HUMAN VITAMIN D RECEPTOR" PROCEEDINGS OF THE NATIONAL ACADEMY OF SCIENCES OF THE UNITED STATES, vol. 85, no. 10, 1988, pages 3294-3298, XP000982874 1988 ISSN: 0027-8424
- GOTO H ET AL: "A SINGLE RECEPTOR IDENTICAL WITH THAT FROM INTESTINE-T47D CELLS MEDIATES THE ACTION OF 1 25 DIHYDROXYVITAMIN D-3 IN HL-60 CELLS" BIOCHIMICA ET BIOPHYSICA ACTA, vol. 1132, no. 1, 1992, pages 103-108, XP000991535 ISSN: 0006-3002
- BRZOZOWSKI A ET AL: "Molecular basis of agonism and antagonism in the oestrogen receptor" NATURE,GB,MACMILLAN JOURNALS LTD. LONDON, no. 389, 16 octobre 1997 (1997-10-16), pages 753-758, XP002075907 ISSN: 0028-0836 cité dans la demande
- HAUSSLER M R ET AL: "THE VITAMIN D HORMONE AND ITS NUCLEAR RECEPTOR: MOLECULAR ACTIONS AND DISEASE STATES" JOURNAL OF ENDOCRINOLOGY,GB,BRISTOL, vol. 154, no. 154, septembre 1997 (1997-09), pages S57-S73-S73, XP000867746 ISSN: 0022-0795
- POULJOL N ET AL: "3D model of the androgen receptor (AR) ligand-binding (LBD): Validation by artificial and naturally occurring mutations of the androgen receptor gene." HORMONE RESEARCH (BASEL), vol. 50, no. SUPPL. 3, 1998, page 7 XP000933935 37th Annual Meeting of the European Society for Paediatric Endocrinology;Florence, Italy; September 24-27, 1998 ISSN: 0301-0163
- DATABASE WPI Section Ch, Week 199907 Derwent Publications Ltd., London, GB; Class B04, AN 1999-080898 XP002146587 & WO 98 56908 A (CHUGAI SEIYAKU KK), 17 décembre 1998 (1998-12-17)
- MANGELSDORF DAVID J ET AL: "The nuclear receptor superfamily: The second decade." CELL, vol. 83, no. 6, 15 décembre 1995 (1995-12-15), pages 835-839, XP000929729 ISSN: 0092-8674 cité dans la demande
- ROCHEL N ET AL: "The crystal structure of the nuclear receptor for vitamin D bound to its natural ligand." MOLECULAR CELL., vol. 5, no. 1, janvier 2000 (2000-01), pages 173-179, XP000929728 ISSN: 1097-2765

## Description

L'invention a pour objet des polypeptides dérivés du récepteur nucléaire de la vitamine D, les séquences nucléotidiques codant pour ces polypeptides, ainsi que l'utilisation de ces polypeptides notamment dans le cadre du criblage d'analogues synthétiques de la vitamine D, ou dans la mise au point de tests (par exemple double ou triple hybride,...) pour l'identification d'autres protéines (activateur, répresseur, ...) interagissant avec le récepteur de la vitamine D par l'utilisation de constructions contenant le polypeptide en fusion avec Gal4 par exemple, ou dans le cadre de l'analyse de la structure tridimensionnelle des complexes formés entre ces polypeptides et une molécule particulière par des techniques de cristallographie ou RMN.

Le récepteur de la vitamine D (VDR) est un régulateur transcriptionnel dépendant du ligand, appartenant à la superfamille des récepteurs nucléaires (NRs) (Mangelsdorf et al., 1995).

Les membres de cette famille possèdent la même structure modulaire avec un domaine de liaison à l'ADN (DBD) hautement conservé et un domaine de liaison au ligand (LBD) davantage variable (Mangelsdorf et al., 1995 ; Wurtz et al., 1996).

Le VDR se lie à l'élément de réponse correspondant, de type DR3, dans la région promotrice des gènes cibles, sous forme d'hétérodimère avec le récepteur X de l'acide rétinoïque (RXR), ce qui conduit à l'activation ou à la répression de la transcription par l'intermédiaire d'une interaction avec les cofacteurs transcriptionnels et la machinerie transcriptionnelle basale (Deluca & Zierold, 1998).

Les métabolites de la vitamine D sont utilisés, ou sont susceptibles d'être utilisés, dans des traitements variés dans le cadre de l'ostéodystrophie, l'ostéoporose, le psoriasis, le cancer et les maladies auto-immunes (Bouillon et al., 1995).

L'hypercalcémie induite par la vitamine D (ou 1α, 25 - dihydroxyvitamine D₃, ou 1,25(OH)₂ D₃) limite l'utilisation du ligand naturel dans ces applications cliniques, ce qui a conduit au développement d'analogues susceptibles de posséder des effets secondaires réduits.

La séquence du LBD de hVDR est faiblement conservée en comparaison avec les séquences du récepteur de l'acide rétinoïque γ humain (hRAR γ) et du récepteur X de l'acide rétinoïque α humain (hRXRα) (respectivement 25 % et 17 % d'identité avec le hVDR).

La présence d'un domaine d'insertion dans le LBD des VDR connectant les hélices H1 et H3, représente une caractéristique typique des VDR. La taille de cette région de connexion varie entre 72 à 81 résidus dans la famille des VDR, tandis qu'elle varie entre 15 à 25 résidus chez les autres récepteurs nucléaires.

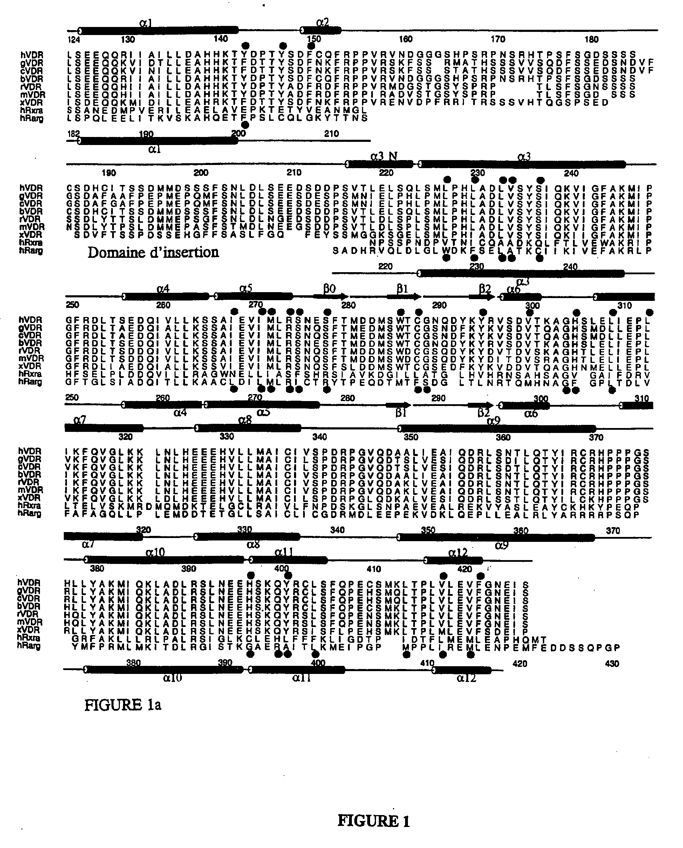

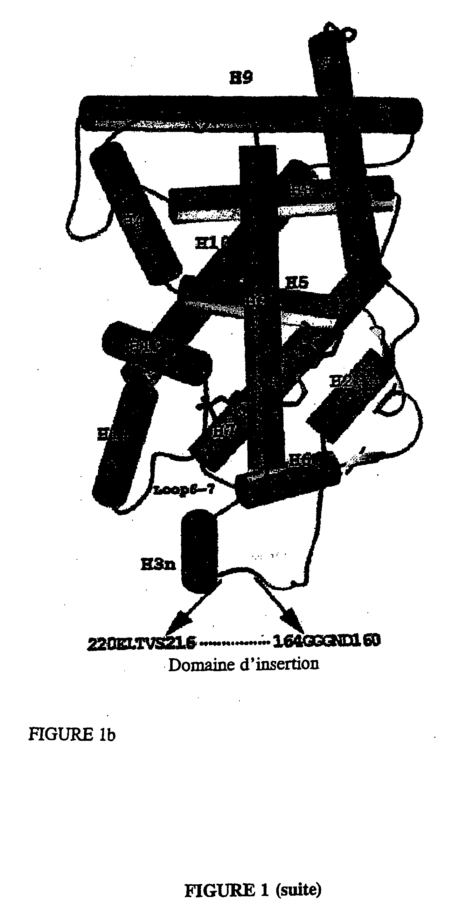

Il convient de souligner que dans ce qui suit la numérotation des acides aminés utilisée pour les séquences peptidiques des différents VDR, correspond à la numérotation des acides aminés du VDR humain. L'extension de cette numérotation aux autres séquences que celle du VDR humain peut se faire sans ambiguïté à partir de l'alignement donné sur la figure 1a ci-après.

Le taux de conservation de séquence de ce domaine d'insertion est très faible (9 % d'identité entre les acides aminés 157-215 du hVDR). Cette région est accessible aux protéases, et contient un site de phosphorylation au niveau de la sérine en position 208, pour lequel aucun rôle fonctionnel n'a pu être défini.

La présence de ce domaine pourrait expliquer les difficultés rencontrées jusqu'à présent pour cristalliser les LBD des VDR. En effet, ce domaine est faiblement structuré comme le montre les analyses de structure secondaire qui ne prédisent que quelques brins courts avec des statistiques assez faibles, et contient un pourcentage très élevé de résidus chargés négativement. Ces deux facteurs pourraient augmenter le nombre de conformères de cette boucle, affectant ainsi la stabilité de la protéine, et favorisant les contacts non spécifiques qui interfèrent avec les processus de cristallisation.

La présente invention a pour but de fournir des polypeptides dérivés des récepteurs nucléaires de la vitamine D sous une forme soluble et pouvant être cristallisés.

L'invention a également pour but de fournir des séquences nucléotidiques codant pour ces polypeptides dérivés, ainsi que des procédés de préparation desdits polypeptides dérivés par transformation de cellules appropriées avec lesdites séquences nucléotidiques.

L'invention a également pour but de fournir de nouvelles méthodes de criblage de composés analogues de la vitamine D et/ou d'analyse de la structure tridimensionnelle des complexes formés entre ces polypeptides et une molécule particulière, lesdites méthodes étant effectuées à l'aide des polypeptides dérivés susmentionnés.

L'invention a également pour but de fournir des kits pour la mise en oeuvre des méthodes susmentionnées.

L'invention sera illustrée à l'aide des figures suivantes :
- Figure 1 : a) alignement des séquences peptidiques des VDR de différentes espèces (hVDR : VDR d'*homo sapiens* [humain]; bVDR : VDR de *bos taurus* [bovin]; gVDR : VDR de *gallus gallus* [poulet]; rVDR : VDR de *ratus norvegicus* [rat]; mVDR : VDR de *mus musculus* [souris]; cVDR : VDR de *cotumic japonica* [caille japonaise]; xVDR : VDR de *xenopus laevi* [grenouille africaine]) avec les séquences du RXRα humain (hRXRα) et du RARγ humain (hRARγ). Les domaines d'insertion des VDR qui ont été supprimés sont ceux encadrés.
   b) conformation générale du domaine de liaison au ligand de hVDR; les hélices sont représentées par des cylindres et les feuillets β par des flèches.

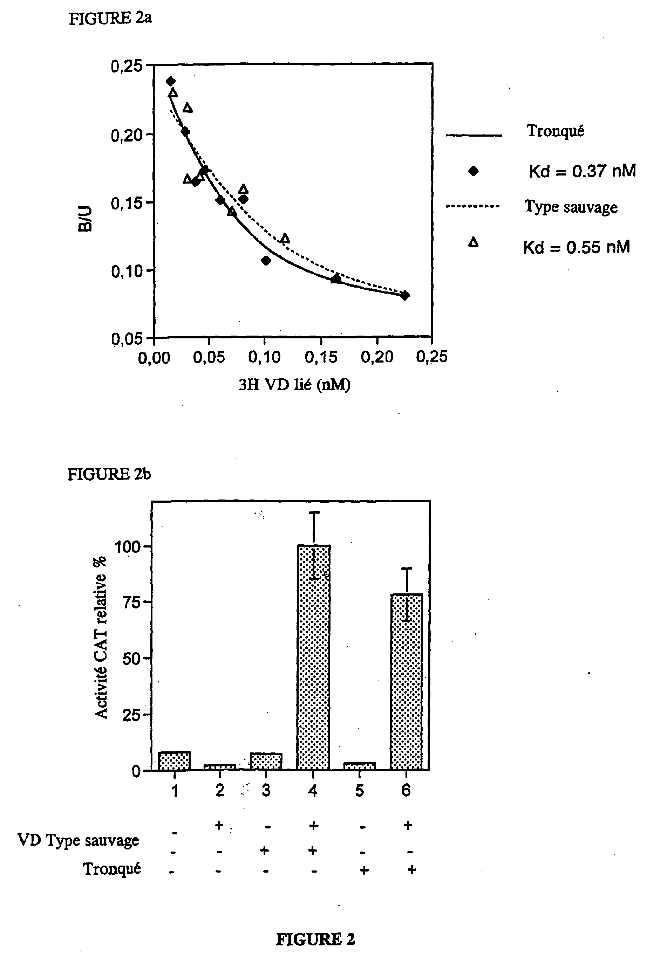

- Figure 2 : a) analyse de Scatchard de la liaison de 1,25(OH)₂ D₃ au LBD du hVDR sauvage (118-427; courbe en pointillé indiquée par des triangles) et au LBD du mutant dérivé du hVDR (118-427 Δ 165-215; courbe pleine indiquée par des losanges) ; la quantité de 1,25(OH)₂ D₃ marquée liée est indiquée en abscisse en nM, et le rapport B/U entre les LBD sauvage ou mutant liés (B) et non liés (U) est indiqué en ordonnée.
   b) activités CAT du hVDR sauvage et du hVDR mutant; ces activités sont indiquées en ordonnée en pourcentage; la colonne 1 correspond à l'activité CAT mesurée en absence de vitamine D (VD), de hVDR sauvage (sauvage) et de hVDR mutant (tronqué); la colonne 2 correspond à l'activité CAT mesurée en présence de VD, et en l'absence de hVDR sauvage et de hVDR mutant; la colonne 3 correspond à l'activité CAT mesurée en présence de hVDR sauvage, et en l'absence de VD et de hVDR mutant; la colonne 4 correspond à l'activité CAT mesurée en présence de VD et de hVDR sauvage, et en l'absence de hVDR mutant; la colonne 5 correspond à l'activité CAT mesurée en présence de hVDR mutant, et en l'absence de VD et de hVDR sauvage; la colonne 6 correspond à l'activité CAT mesurée en présence de hVDR mutant et de VD, et en l'absence de hVDR sauvage.
- Figure 3 : a) représentation de la région du feuillet β de RARγ,
   b) représentation de la région du feuillet β du VDR dans la même orientation que celle de RARγ ci-dessus,
   c) représentation des interactions intramoléculaires de l'hélice H12 dans le VDR,
   d) interface entre le LBD du VDR et l'hélice H3n d'une molécule symétriquement reliée.
- Figure 4 : a) schéma de 1,25(OH)₂ D₃,
   b) représentation schématique de la poche de liaison au ligand du hVDR,
   c) vitamine D dans sa densité électronique contourée à 1σ,
   d) cavité du ligand.

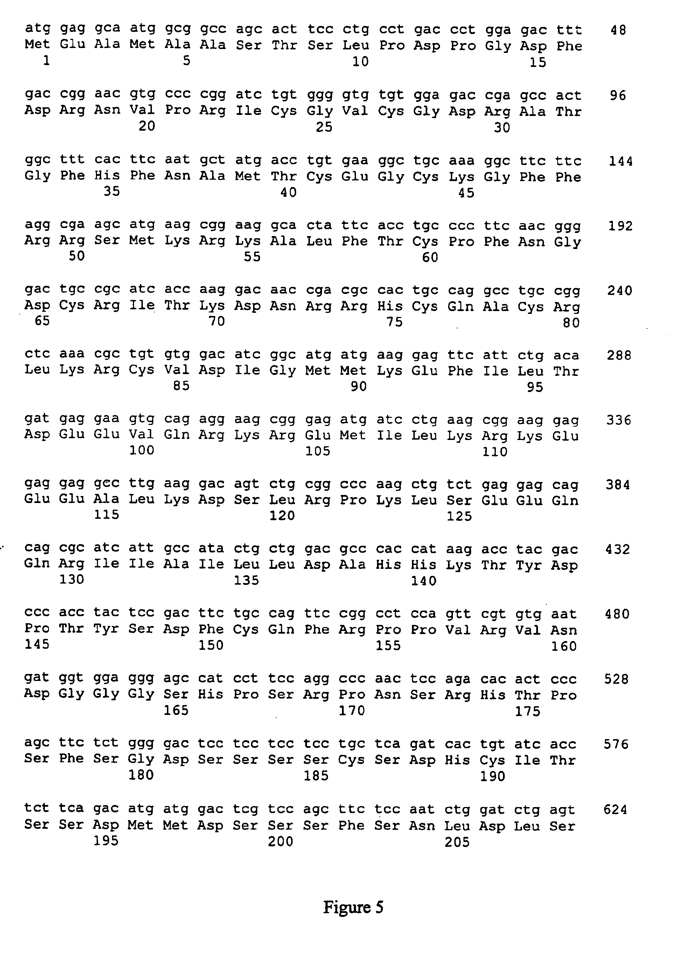

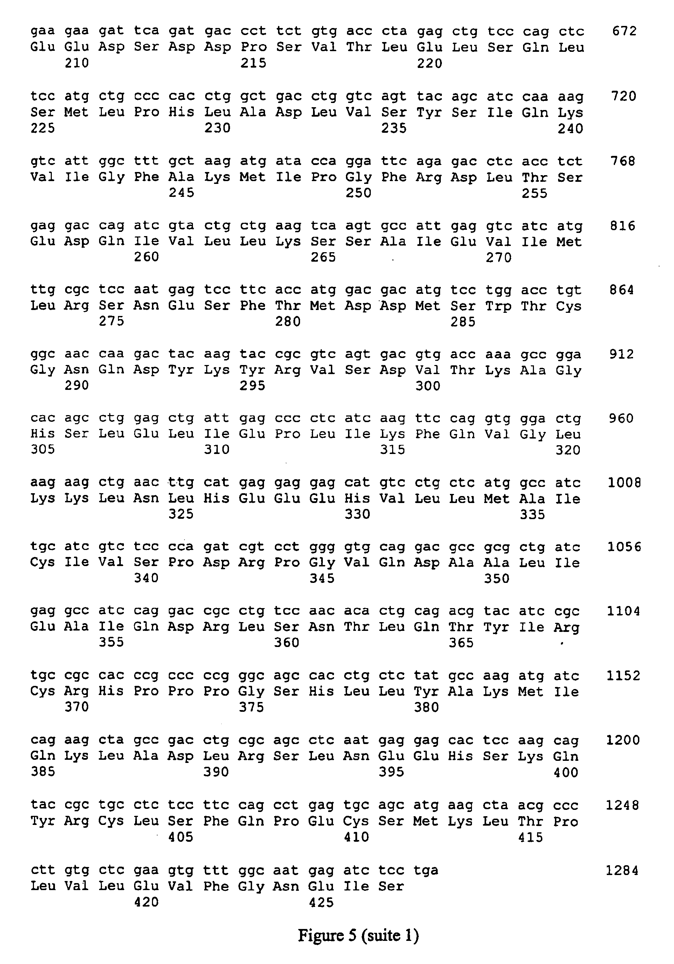

- Figure 5 : séquence nucléotidique codant pour le hVDR sauvage (SEQ ID NO : 1), et séquence peptidique du hVDR sauvage (SEQ ID NO : 2),
- Figure 6 : séquence nucléotidique (SEQ ID NO : 3) codant pour le polypeptide mutant dérivé du hVDR (1-427 Δ 165-215), et séquence peptidique dudit polypeptide (SEQ ID NO : 4),
- Figure 7 : séquence nucléotidique (SEQ ID NO : 5) codant pour le polypeptide mutant dérivé du hVDR (118-427 Δ 165-215), et séquence peptidique dudit polypeptide (SEQ ID NO : 6),
- Figure 8 : séquence nucléotidique (SEQ ID NO : 7) codant pour le polypeptide mutant dérivé du hVDR (124-427 Δ 165-215), et séquence peptidique dudit polypeptide (SEQ ID NO : 8).

La présente invention a pour objet les polypeptides dérivés du récepteur nucléaire de la vitamine D chez l'homme ou les différentes espèces du règne animal possédant un tel récepteur, ledit récepteur nucléaire comprenant un domaine de liaison au ligand, ou LBD, ce LBD contenant un domaine d'insertion flexible, lesdits polypeptides dérivés étant caractérisés en ce qu'ils comprennent :
- les séquences peptidiques desdits récepteurs nucléaires dans lesquelles :
   * le domaine d'insertion flexible du LBD est modifié par substitution ou suppression d'au moins environ 30 acides aminés, et de préférence d'au moins environ 40 acides aminés, ou de la totalité des acides aminés composant ce domaine d'insertion (à savoir environ 50 ± 10 acides aminés),
   * et, le cas échéant, un ou plusieurs ou la totalité des acides aminés situés aux positions 1 à environ 125, notamment aux positions 1 à 117 ou 123, des séquences peptidiques desdits VDR, sont modifiés par substitution ou suppression,
   lesdits polypeptides dérivés possédant les caractéristiques suivantes :
   * les propriétés de liaison au ligand et de transactivation du LBD du récepteur de la vitamine D sont conservées,
   * ils sont stables, à savoir qu'ils peuvent être conservés, notamment dans NaCl 100 mM à pH 7 pendant au moins environ une semaine, sans que soit pour autant affectées les propriétés susmentionnées du LBD, par opposition au LBD non modifié qui est instable dans les conditions susmentionnées,
   * ils sont cristallisables dans des solvants aqueux, notamment à 4°C par la méthode de diffusion de vapeur à goutte suspendue,
   * et, ils sont solubles dans des solvants aqueux,
- ou les séquences peptidiques dérivées des séquences peptidiques définies ci-dessus, notamment par suppression, addition ou substitution d'un ou plusieurs acides aminés, lesdites séquences dérivées possédant les caractéristiques mentionnées ci-dessus desdits polypeptides dérivés.

L'invention a plus particulièrement pour objet les polypeptides dérivés des VDR d'origine humaine ou animale tels que définis ci-dessus, lesdits polypeptides dérivés étant caractérisés en ce qu'ils comprennent les séquences peptidiques desdits récepteurs nucléaires dans lesquelles est supprimé le fragment peptidique délimité :
- d'une part, par un acide aminé situé approximativement à l'une des positions 155 à 175 des séquences peptidiques des récepteurs nucléaires de la vitamine D d'origine humaine ou animale, notamment des séquences peptidiques des VDR représentées sur la figure 1a, et plus particulièrement par l'acide aminé situé à l'une des positions 159 à 168 de ces séquences,
- et, d'autre part, par un acide aminé situé approximativement à l'une des positions 204 à 225 des séquences peptidiques des récepteurs nucléaires de la vitamine D d'origine humaine ou animale, notamment des séquences peptidiques des VDR représentées sur la figure 1a.

L'invention concerne plus particulièrement les polypeptides dérivés tels que définis ci-dessus, lesdits polypeptides dérivés étant choisis parmi ceux comprenant :
- la séquence en acides aminés délimitée par les acides aminés situés aux positions 118 et 427, ou aux positions 124 et 427 des séquences peptidiques des VDR d'origine humaine ou animale, notamment des séquences peptidiques des VDR représentées sur la figure 1a, et dans laquelle les résidus situés aux positions 165 à 215 desdites séquences peptidiques des VDR sont supprimés,
- ou une séquence peptidique dérivée de la séquence en acides aminés susmentionnée, notamment par suppression, addition ou substitution d'un ou plusieurs acides aminés, ladite séquence dérivée possédant les caractéristiques mentionnées ci-dessus dudit polypeptide dérivé.

L'invention concerne plus particulièrement les polypeptides dérivés tels que définis ci-dessus choisis parmi les suivants :
- le polypeptide SEQ ID NO : 4 dérivé du VDR d'origine humaine dans lequel le fragment peptidique délimité par les acides aminés situés aux positions 165 et 215 est supprimé,
- le polypeptide SEQ ID NO : 6 [encore désigné hVDR (118-427 Δ 165-215)] dérivé du VDR d'origine humaine dans lequel le fragment peptidique délimité par les acides aminés situés aux positions 1 et 117, et le fragment peptidique délimité par les acides aminés situés aux positions 165 et 215 sont supprimés,
- le polypeptide SEQ ID NO : 8 [encore désigné hVDR (124-427 Δ 165-215)] dérivé du VDR d'origine humaine dans lequel le fragment peptidique délimité par les acides aminés situés aux positions 1 et 123, et le fragment peptidique délimité par les acides aminés situés aux positions 165 et 215 sont supprimés.

L'invention concerne également les séquences nucléotidiques codant pour un polypeptide dérivé tel que défini ci-dessus.

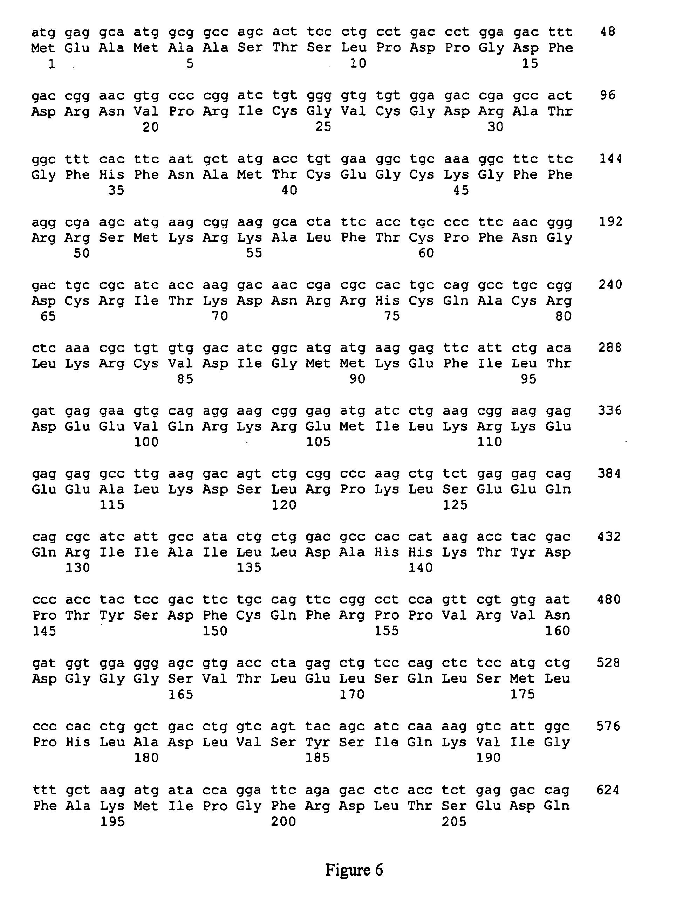

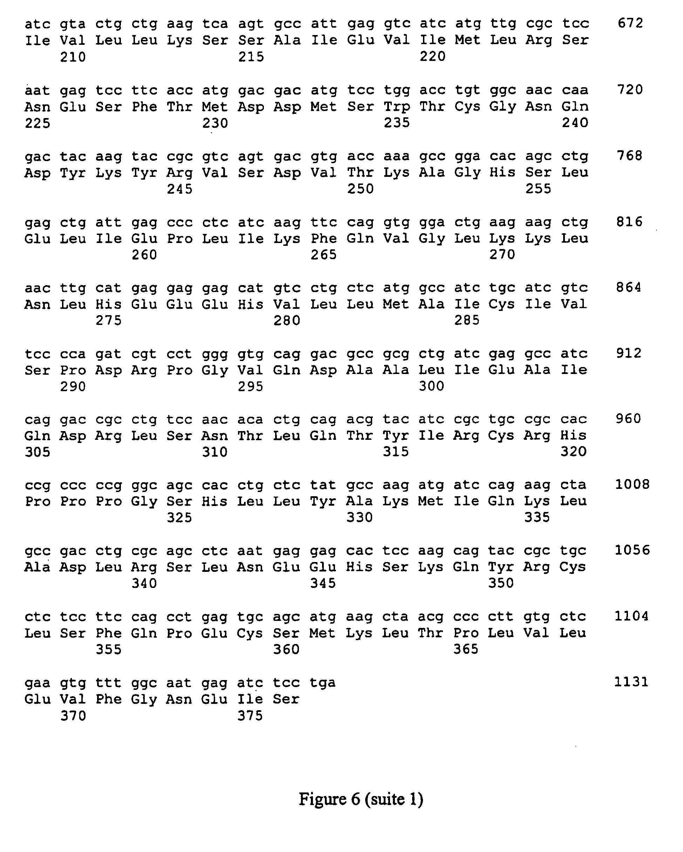

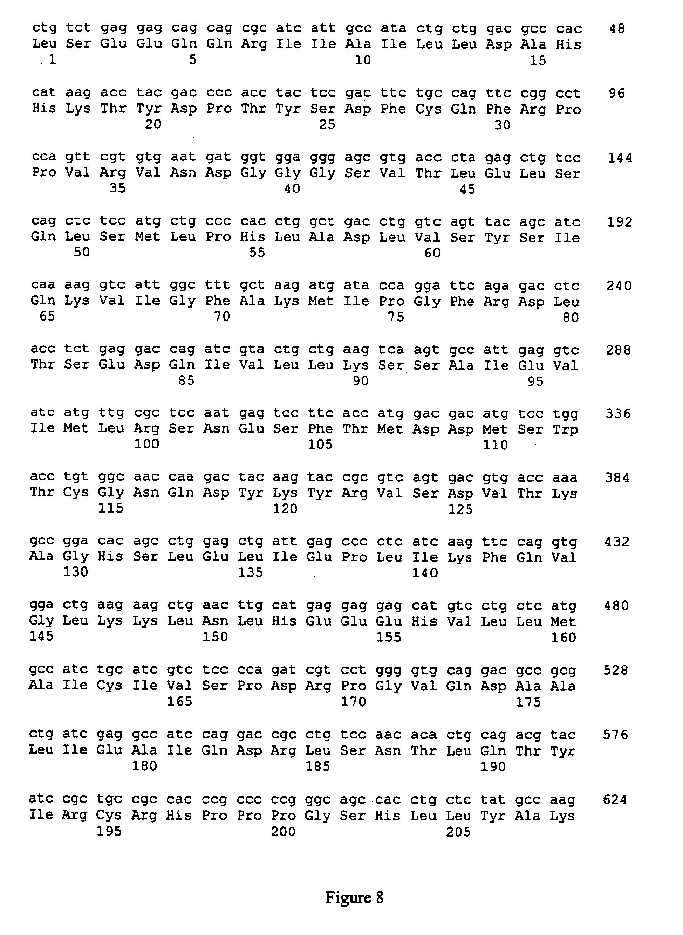

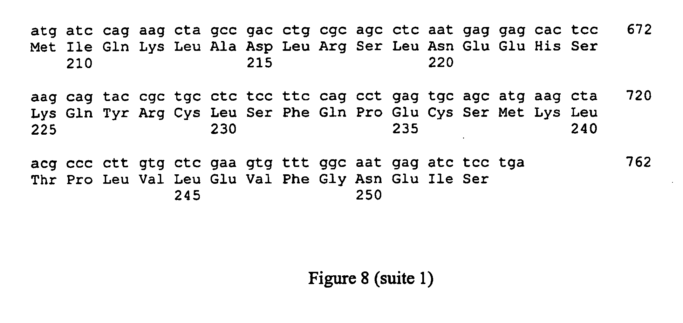

A ce titre, l'invention a plus particulièrement pour objet les séquences nucléotidiques choisies parmi :
- les séquences SEQ ID NO : 3, SEQ ID NO : 5 et SEQ ID NO : 7, représentées respectivement sur les figures 6, 7, et 8,
- ou une séquence nucléotidique dérivée par dégénérescence du code génétique des séquences nucléotidiques susmentionnées, et codant pour un polypeptide dérivé du HVDR tel que défini ci-dessus,
- ou une séquence nucléotidique dérivée des séquences nucléotidiques susmentionnées, notamment par substitution, suppression ou addition d'un ou plusieurs nucléotides, et codant pour une séquence peptidique dérivée des polypeptides dérivés du hVDR définis ci-dessus, et possédant les caractéristiques mentionnées ci-dessus desdits polypeptides dérivés.

L'invention a également pour objet les séquences nucléotidiques recombinantes comprenant une séquence nucléotidique telle que définie ci-dessus en association avec les éléments nécessaires à la transcription de cette dernière séquence, notamment avec un promoteur et un terminateur de transcription.

L'invention concerne également les vecteurs, notamment plasmides ou virus tels que les baculovirus, contenant une séquence nucléotidique telle que définie ci-dessus.

L'invention a également pour objet les cellules hôtes transformées par un vecteur susmentionné, lesdites cellules étant choisies notamment parmi les bactéries telles que *E. coli*, ou les cellules d'insectes susceptibles d'être infectées par un baculovirus.

L'invention concerne également un procédé de préparation d'un polypeptide dérivé tel que défini ci-dessus, ledit procédé comprenant les étapes suivantes :
- transformation de cellules à l'aide d'un vecteur recombinant tel que défini ci-dessus,
- mise en culture des cellules ainsi transformées, et récupération dudit polypeptide produit par lesdites cellules, le cas échéant après purification.

L'invention concerne également les polypeptides dérivés du récepteur nucléaire de la vitamine D tels que définis ci-dessus, liés à la vitamine D ou à un analogue de la vitamine D, notamment à tout ligand capable de se lier auxdits polypeptides avec une haute affinité, à savoir une affinité supérieure à environ 10⁻⁶ M.

L'invention concerne également les polypeptides dérivés du récepteur nucléaire de la vitamine D tels que définis ci-dessus, le cas échéant liés à la vitamine D ou à un analogue de la vitamine D, et se présentant sous forme de cristaux.

Avantageusement, les cristaux susmentionnés de la présente invention sont obtenus par diffusion de vapeur en présence notamment de sulfate d'ammonium en tant que précipitant, ou d'un autre agent précipitant.

Avantageusement encore, les cristaux de l'invention sont utilisables dans le cadre de techniques de cristallographie aux rayons X.

Les cristaux susmentionnés peuvent atteindre une résolution déterminée par cristallographie aux rayons X inférieure à 25 Å, qui fournit une information au niveau atomique de l'interaction entre une molécule cible et le récepteur.

L'invention a plus particulièrement pour objet les cristaux de hVDR (118-427 Δ 165-215) complexé à 1,25 (OH)₂D₃), caractérisés en ce qu'ils appartiennent au groupe d'espace orthorhombique (P2₁ 2₁2₁) avec a = 45.193 Å, b = 52.443 Å, c = 133.286 Å, &=β=γ=90°.

L'invention a également pour objet l'utilisation d'un polypeptide tel que défini ci-dessus, le cas échéant sous forme de cristaux susmentionnés, pour la mise en oeuvre d'une méthode de criblage d'analogues synthétiques de la vitamine D.

L'invention a plus particulièrement pour objet l'utilisation susmentionnée d'un polypeptide tel que défini ci-dessus, le cas échéant sous forme de cristaux susmentionnés, pour la mise en oeuvre d'une méthode de criblage d'analogues agonistes ou antagonistes de la vitamine D susceptibles d'être utilisés dans des compositions pharmaceutiques, notamment dans le cadre du traitement de pathologies cancéreuses, de l'ostéodystrophie, de l'ostéoporose, du psoriasis, des maladies auto-immunes.

L'invention concerne également des procédés de criblage d'analogues de la vitamine D, ou de cofacteurs, comprenant les étapes suivantes :
- mise en présence d'un polypeptide dérivé tel que défini ci-dessus, le cas échéant sous forme de cristaux susmentionnés, avantageusement lié à un support solide, avec l'analogue ou cofacteur testé, l'un dudit polypeptide dérivé ou de l'analogue de la vitamine D étant avantageusement marqué, notamment à l'aide d'un marqueur fluorescent, radioactif ou enzymatique,
- détection de l'éventuelle liaison entre ledit polypeptide dérivé et l'analogue testé par mesure du marqueur utilisé, notamment après rinçage du support utilisé lors de l'étape précédente.

L'invention a également pour objet l'utilisation d'un polypeptide dérivé tel que défini ci-dessus, le cas échéant sous forme de cristaux susmentionnés, pour la mise en oeuvre d'une méthode d'analyse de la structure tridimensionnelle des complexes formés entre ledit polypeptide et une molécule déterminée.

A ce titre l'invention concerne plus particulièrement une méthode d'analyse de la structure tridimensionnelle des complexes formés entre un polypeptide dérivé tel que défini ci-dessus, le cas échéant sous forme de cristaux susmentionnés, et une molécule déterminée, ledit procédé comprenant les étapes suivantes :
- mise en présence d'un polypeptide dérivé tel que défini ci-dessus, le cas échéant sous forme de cristaux susmentionnés, avec la molécule déterminée,
- cristallisation du complexe formé entre ledit polypeptide dérivé et la molécule déterminée, notamment par diffusion de vapeur, et analyse tridimensionnelle dudit complexe notamment par remplacement moléculaire,
- ou analyse tridimensionnelle dudit complexe en solution, notamment par RMN.

L'invention concerne également l'application - de la méthode d'analyse susmentionnée à la conception de composés susceptibles d'être des agonistes ou des antagonistes de la vitamine D tels que définis ci-dessus.

L'invention a plus particulièrement pour objet les analogues agonistes ou antagonistes de la vitamine D tels qu'obtenus par mise en oeuvre d'un procédé de criblage susmentionné, ainsi que les compositions pharmaceutiques comprenant ces analogues en association avec un véhicule pharmaceutiquement acceptable.

L'invention a également pour objet les kits (ou trousses) pour la mise en oeuvre d'un procédé de criblage ou d'une méthode d'analyse susmentionnés, lesdits kits comprenant un polypeptide dérivé tel que défini ci-dessus, le cas échéant sous forme de cristaux susmentionnés, en association avec un ou plusieurs réactifs pour la mise en oeuvre du procédé ou de la méthode susmentionnée.

L'invention sera davantage illustrée à l'aide de la description détaillée qui suit de la préparation du polypeptide dérivé hVDR (118-427 Δ 165-215), et de l'analyse de la structure cristalline et des propriétés du polypeptide dérivé ainsi obtenu.

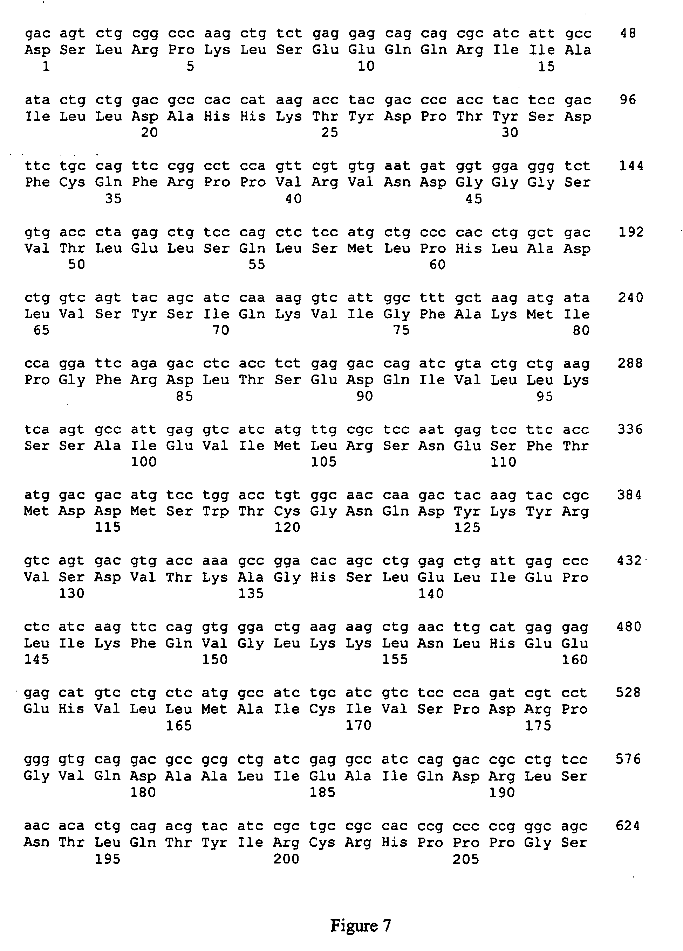

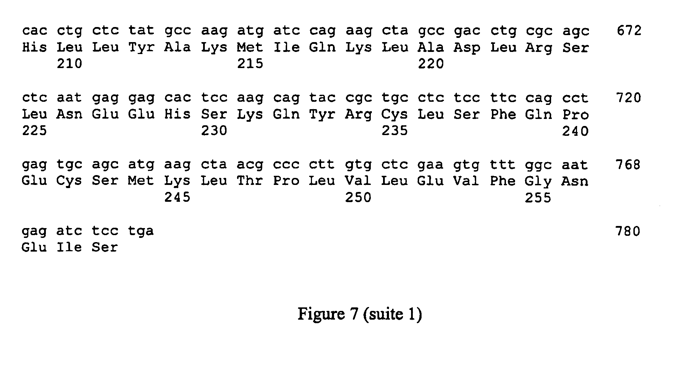

Le polypeptide dérivé représenté sur la figure 7 (encore désigné mutant LBD VDR (résidus 118-427 Δ 165-215)), a été préparé sans son domaine d'insertion flexible en supprimant les résidus 165 à 215 de hVDR (Figure 1a) et en laissant une trentaine de résidus afin de connecter les hélices H1 et H3.

Le mutant LBD VDR (résidus 118-427 Δ 165-215) a été surexprimé dans E. coli et purifié par chromatographie d'affinité et d'échange d'ions, et par filtration sur gel, selon la méthode décrite ci-après.

### A) Expression et purification du mutant LBD VDR 118-427 Δ 165-215

Le récepteur VDR muté (résidus 118-427 Δ 165-215) a été surproduit sous forme de peptide avec un tag hexahistidine. Le cDNA amplifié par PCR a été sous-cloné dans les sites NdeI-BamHI du vecteur pET15b (Novagen). Le plasmide a été ensuite amplifié dans les bactéries *E. coli* XL-1 Blue afin de contrôler la séquence et a été introduit dans les bactéries *E. coli* BL21DE3 pour la surexpression. Une préculture de 200 ml de LB avec 200 µg ampicilline/ml est utilisée pour inoculer 6x 11 de LB contenant 200 µg ampicilline/ml: Les cellules sont cultivées à 37°C jusqu'à une absorbance de 0.6 puis l'expression de la protéine est induite par l'addition de 1 mM d'IPTG dans le milieu de culture durant 6h à 20°C. Les cellules sont culottées par ultracentrifugation et conservées à -80°C.

Le culot cellulaire représentant 1 1 de culture est repris dans 25 ml de tampon contenant 20mM Tris pH8.0, 250 mM NaCl, 5 mM Imidazole, 5% glycérol, 0.5 µg/ml du cocktail d'inhibiteur de protéases, 1 mM de β-mercaptoéthanol et 1 mM de PMSF. La lyse des cellules s'effectue par sonication et l'extrait brut est obtenu par ultracentrifugation à 45 K pendant 1h30. La purification s'effectue en trois étapes. L'extrait brut est dans premier temps chargé sur une colonne d'affinité métallique (Talon, Clonetech). Après lavage, la protéine est éluée avec un tampon contenant 20 mM Tris pH8.0, 250 mM NaCl, 150 mM Imidazole et 5% glycérol. La protéine est ensuite concentrée sur Centriprep 30 et diluée avec 4 volumes de tampon 20 mM Tris ph7.5, NaCl 50mM et DTT 5 mM. L'échantillon est ensuite chargé sur une colonne d'échange anionique Q15 (Sartorius) et élué par un gradient de NaCl (0→1M). Afin de couper le tag hexahistidine, la protéine est digérée par la thrombine (1 unité par mg de protéine) à 4°C pendant 12h en présence de CaCl2 5mM. Finalement la protéine est chargée sur gel de filtration Superdex 75 16/60 (Pharmacia) équilibrée avec 10 mM Tris pH7.0, 100 mM NaCl, 10 mM DTT et éluée avec ce même tampon. Le ligand est alors ajouté en excès et incubé avec la protéine à 4°C durant 12h. Le complexe est alors concentré sur Centricon 30 pour la cristallisation.

La quantité de protéine purifiée est de 2 mg/l de culture. La qualité et l'homogénéité de la protéine sont analysées par électrophorèse en condition dénaturante et native. La protéine est pure à plus de 95% et une bande unique est observée sur gel natif. La protéine est monomérique selon l'élution de la filtration sue gel et des mesures de diffusion de lumière. La concentration en protéine est mesurée par Bradford et par spectrophotométrie. L'échantillon est monodisperse selon les mesures de diffusion de lumière.

### B) Analyse et propriétés du VDR 118-427 Δ 165-215.

La capacité de la protéine mutante à se lier à 1,25(OH)₂D₃ a été déterminée par la méthode de Scatchard en utilisant des extraits bruts de la protéine recombinante (Figure 2a : les analyses de Scatchard ont été effectuées sur Dextran/charbon; les extraits bruts de *E. coli* BL21 (DE3) exprimant hVDR sauvage ou mutant / pET 15b, ont été dilués 1000 fois et incubés avec des quantités croissantes (³H-26,27, Amersham) 1,25(OH)₂D₃ dans Tris 20 mM, NaCl 250 mM, dithiothréitol (DTT) 5 mM, glycérol 10% pendant 16 heures à 4°C; après incubation, 25 µl de Dextran/charbon (1,5%) ont été additionnés à 25 µl du mélange de protéines; après 5 min les tubes ont été centrifugés à 13000 rpm pendant 5 min; les concentrations de ligand lié (B) ont été déterminées par comptage en scintillation liquide sur le surnageant; les concentrations en ligand total ont été mesurées par comptage en scintillation liquide sur 15 µl du mélange de protéines avant addition de Dextran/charbon; U représente le ligand non lié; chaque point représente la moyenne de trois valeurs; les résultats ont été analysés par la méthode non linéaire de moindre carré selon la méthode décrite par Claire et al., 1978; la courbe pleine et celle en pointillé correspondent respectivement aux résultats expérimentaux obtenus avec le polypeptide mutant dérivé et la protéine sauvage, avec les paramètres N = 0,073 ± 0,006 nM, Kd = 0,37 ± 0,05 nM, β = 0,058 ± 0,002 pour le polypeptide mutant dérivé, et N = 0,10 ± 0,01 nM, Kd = 0,55 ± 0,08 nM, β = 0,051 ± 0,003 pour la protéine sauvage, avec N = nombre de sites, Kd = constante de dissociation, et β = liaison non spécifique; les expériences ont été répétés deux fois).

Aucun changement significatif entre les constantes de dissociation des VDR du type sauvage et mutant n'a été observé, les valeurs étant similaires à celle décrites préalablement pour le récepteur entier (Bouillon et al., 1995). Afin de comparer les propriétés de transactivation des deux protéines, les LBD sauvages et mutants ont été fusionnées au domaine de liaison à l'ADN de l'activateur GAL4 de la levure. Les protéines chimériques ont été exprimées par transfection dans les cellules Cos, et la transactivation a été mesurée avec un rapporteur approprié répondant à GAL4. Les deux protéines présentent des propriétés de transactivation comparables dans ce système (Figure 2b : les LBD de VDR sauvage ou mutant ont été fusionnés au domaine de liaison à l'ADN de l'activateur Gal4 (1-147) de la levure par clonage de l'ADNc dans les sites XhoI-BamHI du vecteur PXJ440 (Xiao et al., 1991); les cellules Cos ont été transfectées selon la méthode décrite par Xiao et al., 1991, avec les vecteurs (250 ng) contenant les LBD sauvage ou mutant de hVDR avec 2 µg de gène rapporteur 17m5-TATA-CAT et 2 µg d'un recombinant de contrôle interne pCH110lacZ (Pharmacia) exprimant la β-galactosidase complété à 20 µg avec un ADN support; les cellules ont été traitées avec EtOH ou 1,25(OH)₂D₃ 10⁻⁷M; les activités CAT, normalisées en unités égales de β-galactosidase, sont exprimées par rapport à l'activité CAT (100%) induite par le VDR sauvage en présence de 1,25(OH)₂D₃).

Par conséquent, la délétion du domaine d'insertion n'a pas d'effet majeur sur la liaison du ligand, la transactivation ou la dimérisation avec le LBD de RXRα.

La suppression du domaine d'insertion flexible dans le LBD de VDR a conduit à une protéine davantage soluble, qui peut être cristallisée sous forme de complexe avec 1,25(OH)₂D₃. Des cristaux ont pu être obtenus par des techniques de diffusion de vapeur en utilisant du sulfate d'ammonium en tant que précipitant. La structure du cristal a été résolue par une combinaison de remplacement moléculaire, en utilisant un modèle partiel de RARγ (Renaud et al. 1995 ; Klaholz et al., 1998), et de remplacement isomorphe avec un dérivé du mercure. Les résultats obtenus sont résumés sur le tableau I ci-après.

**Tableau 1**

| | | | Composé naturel | Dérivé thiomersal |
|---|---|---|---|---|
| **groupe de données** | | | | |
| | source de rayons X | | Hambourg BW7B | Laboratoire |
| | longueur d'onde | | 0,8345 Å | 1,5418 Å |
| | résolution | | 20,0-1,8 Å | 20,0-2,9 Å |
| | reflexions uniques | | 29434 | 6404 |
| | complétude | | 97,4% | 84,9% |
| | multiplicité | | 4,1 | 2,8 |
| | Rsym^{a} | | 6,1% | 9,8% |
| | dernière tranche de résolution | | 24%, 5,06, 93,3% | 20,6%, 3,2, 71,4% |
| | nombre de sites | | | 4 |
| | pouvoir de phasage (c/a) | | | 1,33/1,72 |
| | facteur centrique R | | | 53 % |

| **Affinement** | | | | |
|---|---|---|---|---|
| | facteur R libre (10% des reflexions)^{b} | | 21,4% | |
| | facteur R | | 19,1 % | |
| | nombre d'atomes non hydrogène | | | |
| | | protéine | 1994 | |
| | | ligand | 30 | |
| | | molécules d'eau | 166 | |
| | Rmsd sur la longueur de liaison (Å) | | 0,004 | |
| | Rmsd sur les angles de liaison (Å) | | 1,083 | |

| moyenne des facteurs B pour les atomes non hydrogène (Å²) | | | | |
|---|---|---|---|---|
| | protéine | | 31,,0 | |
| | ligand | | 22,3 | |
| | eau | | 45,6 | |

(Tableau 1 : les expériences de cristallisation ont été effectuées à 4°C par la méthode de diffusion de vapeur à goutte suspendue; la protéine a été concentrée de 4 à 10 mg/ml; les cristaux de hVDR (118-427 Δ 165-215) complexés à 1,25 (OH)₂D₃ ont été obtenus en 4 jours à partir d'une solution contenant du sulfate d'ammonium 0,7 M, tampon Mes 50 mM pH6.0, Tris 5mM, DTT 5mM équilibré contre un réservoir contenant du sulfate d'ammonium 1,4 M, Mes 0,1 M pH = 6.0; les cristaux appartiennent au groupe d'espace orthorhombique (P2₁ 2₁ 2₁) avec a = 45.193 Å, b = 52.443 Å , c = 133.286 Å, α = β = γ = 90° ; l'unité asymétrique contient un monomère; le contenu en solvant des cristaux est de 48 %; le facteur B estimé par la méthode de Wilson est 29. Le dérivé à atome lourd a été obtenu en trempant les cristaux dans du Thiormesal (Ethyl mercurylthiosalicylate) pendant 4 jours; les résultats de diffusion aux rayons X des cristaux naturels ont été mesurés à 4 °C sur la ligne de faisceau BW7B au synchrotron de Hambourg; les résultats ont été traités en utilisant les logiciels DENZO et SCALEPACK (Otwinoswski et al., 1997); les phases initiales ont été obtenues par remplacement moléculaire avec AMORE (Navaza et al., 1994), en utilisant le RAR_{γ} comme modèle de départ; ce modèle contient les hélices conservées H1, H3-H5, H7-H10 (Figure 1a); la solution a une corrélation de 31 % et un facteur R de 53,6 % après remplacement de corps rigide AMORE; complétés par les phases obtenues à l'aide du dérivé mercuriel dont les données de diffraction ont été enregistrées sur un détecteur d'image bidimensionnelle Mar Research au laboratoire à 4° C; les sites dérivés ont été trouvés et affinés avec le logiciel SOLVE (Tervilliger et al., 1987).

La carte obtenue par des phases combinées a été calculée à une résolution de 3 Å en utilisant une correction de solvant. L'affinement a été effectué avec le logiciel CNS (Brünger et al., 1998). Des cycles de construction du modèle avec le logiciel O (Jones et al., 1991) et une minimisation de moindre carré, suivi d'un affinement anisotropique individuel de facteur B ont conduit au modèle final. Tous les résultats entre 20 et 1,8 Å ont été inclus dans l'affinement sans seuils de coupure. Les molécules de solvant de la première couche d'hydratation ont été localisées avec le logiciel CNS sur une carte F₀-F_{c} à 3 sigma. Les valeurs maximales de facteur B (40-50) correspondent aux résidus dans la boucle avant H1, la jonction et les boucles H9-H10 et H11-H12. La qualité du modèle final a été analysée par le logiciel PROCHECK (Laskowski et al., 1993). La distribution d'angle Ramachadran indique 92,4 % de conformations les plus favorables, et 7,6 % de conformations autorisées.

Le modèle final contient 250 résidus, 166 molécules d'eau et une molécule de ligand. Aucune densité claire n'a été observée pour les deux premiers résidus N-terminaux et les quatre derniers résidus C-terminaux. Trois résidus additionnels, 375 à 377 dans la boucle H9-H10, n'ont pas été inclus dans l'affinement en raison de la qualité modérée de la carte de densité électronique dans cette région.

La topologie globale du LBD de VDR (figure 1b) est similaire à celle des autres LBD de récepteurs nucléaires, avec 13 hélices prises en sandwich dans 3 couches et un feuillet β à trois brins. La nomenclature est fondée sur la structure de hRXRα (Bourguet et al., 1995). Le domaine reliant les hélices H1 et H3 contient deux petites hélices H2 et H3n. La nouvelle hélice H3n forme le fond de la structure et remplace la boucle Ω dans la structure de RARγ. La flexibilité intrinsèque des trois résidus glycine (162-164) situés à la jonction favorise une adaptation douce.

Ce fragment de trois résidus est plutôt éloigné du ligand, et il est par conséquent peu probable qu'il joue un rôle dans la liaison au ligand. La structure VDR est plus proche de celle du RARγ complexé à des agonistes. Les protéines se superposent avec un rmsd de 1,2 Å sur 179 résidus (seuil de coupure de 2,5 Å sur Cα), les régions exclues étant le peptide connectant H1 à H3, le brin β2, l'hélice H6 et quelques boucles de connexion.

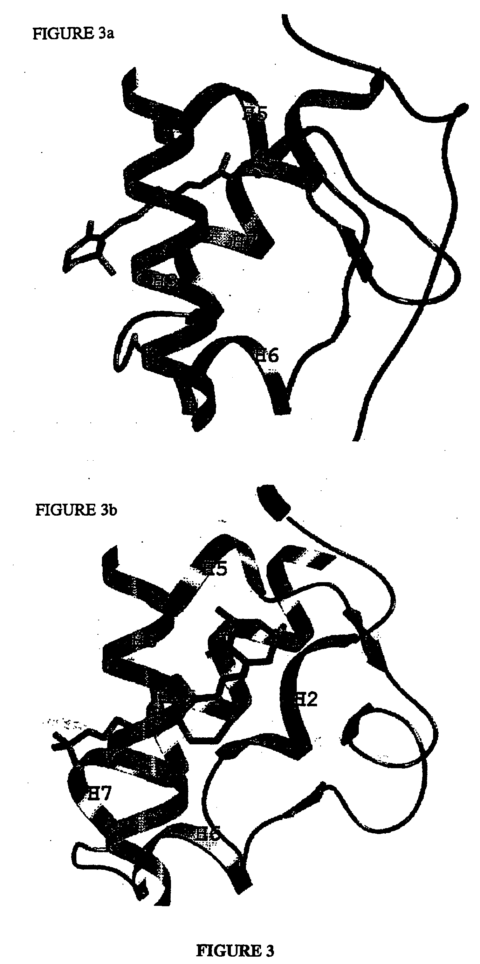

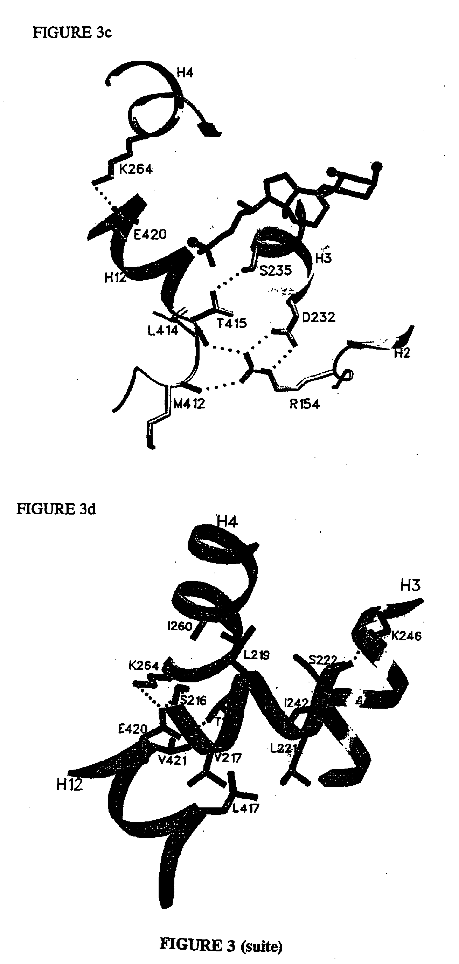

La différence la plus notable se situe au niveau de la connexion entre les hélices H1 et H3, qui dans le RARγ entoure le feuillet β, et dans le VDR suit un chemin entre H3 et l'extrémité du feuillet β, identique à celui de ERα (Brzozowski et al., 1997). Par conséquent, l'extrémité du feuillet β est déplacée vers l'extérieur et agrandit la cavité de liaison à la vitamine D (Figure 3a et 3b). Tous les brins β ont des résidus en contact avec le ligand. Dans le brin β1, Trp 286, spécifique des récepteurs VDR, joue un rôle important dans le positionnement du ligand. Il fait partie d'un réseau de liaisons hydrogène impliquant Ser 275 qui est elle-même liée par liaison hydrogène à Gln 317 et au carbonyle de Met 272. L'extrémité du feuillet β est aussi stabilisée par des liaisons hydrogènes entre les groupes carbonyles de Glu 292 et Lys 294, et Arg 158 de la boucle de connexion entre les hélices H2 et H3n.

L'hélice H12, dont le positionnement est important pour la liaison de coactivation et la transactivation, est en position agoniste (Figure 3c). L'hélice est stabilisée dans cette orientation par des contacts hydrophobes (Ile 268 de H5 et Phe 422 de H12) et interactions polaires. Dans ces dernières interactions sont engagées le pont conservé Lys 264-Glu420, et une liaison hydrogène entre Ser 235 de H3 et Thr 415. De plus, les groupes carbonyles de Met 412 et de Leu 414 de la boucle H11-H12 sont liés par liaison H à Arg 154 (extrémité de H12) qui fait lui-même des liaisons H avec Asp 232 (H3). Tous ces résidus sont conservés chez les récepteurs VDR. La mutagenèse dirigée des résidus Lys 264 et Glu 420 qui établissent le pont salin, a permis de révéler que ces mutations suppriment la transactivation dépendante du ligand, mais n'a pas d'effet sur la liaison au ligand, l'hétérodimérisation avec RXR ou la liaison à l'ADN (Nakajima et al., 1998).

Un fort contact cristallin est observé entre l'hélice H3n et les hélices H3, H4 et H12 d'une molécule symétriquement reliée (Figure 3d). H3n mime les contacts du peptide SRC1 observés dans le complexe ternaire de PPAR (Nolte et al., 1998), bien qu'il ne contienne pas le motif LXXLL trouvé chez la plupart des coactivateurs. Cette hélice est ancrée par contacts polaires à travers des liaisons H entre la région N-terminale de H3n (Ser 216, Val 217 et Thr 218) et Glu 420 (H12). Au niveau de la région C-terminale de l'hélice H3n, Ser 222 est lié par liaison hydrogène à Lys 246 (extrémité de l'hélice H3). L'importance fonctionnelle de cette lysine a été démontrée par mutation en glycine qui affecte fortement la transactivation (Whitfield et al., 1995). Entre les résidus Lys 246 et Glu 420, une cavité hydrophobe est formée par les hélices H3, H4 et H12. Les résidus de l'hélice H3n (Val 217, Thr 218, Leu 219, Leu 221, et Ser 222) sont en contact par liaison van der Waals avec Ile 242 (H3), Ile 260 (H4), Leu 417 et Val 421 (H12). Cette observation montre que d'autres séquences que LXXLL doivent être prises en considération dans le cadre du processus de reconnaissance des récepteurs nucléaires.

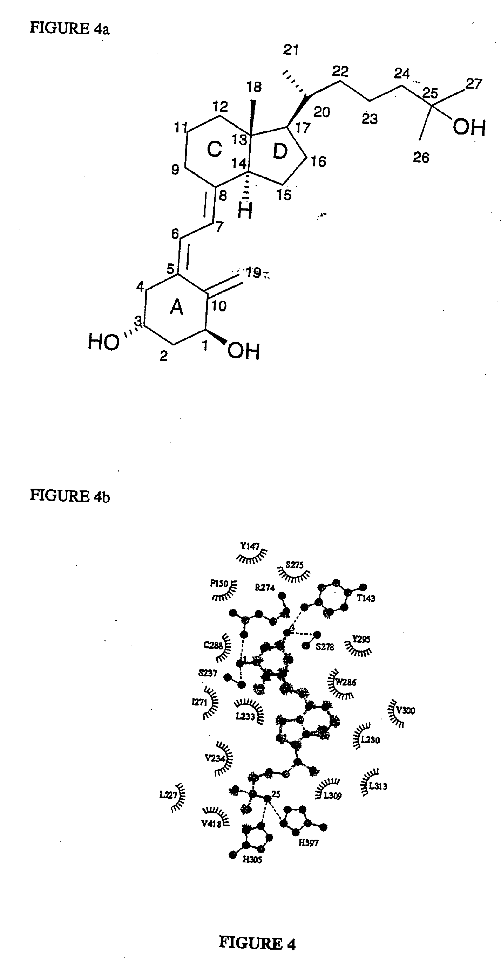

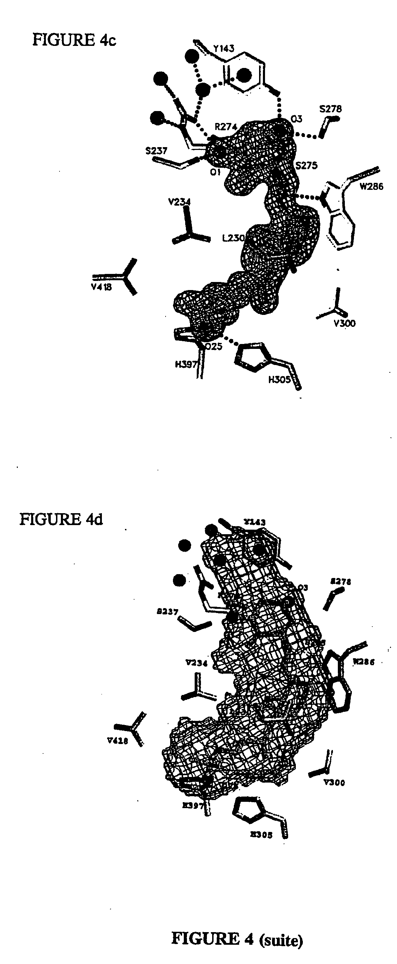

La structure du VDR lié à son ligand naturel a permis de résoudre nombre d'ambiguïtés et de questions concernant la conformation de la vitamine D active. La comparaison avec la structure cristallographique de la molécule libre de vitamine D (Suwinska et al., 1996) montre que les deux ligands ont une conformation identique pour les noyaux A, C et D (Figure 4a). La différence la plus appréciable est la géométrie non planaire du triène conjugué dans le complexe, qui résulte de la forme courbe du ligand nécessaire à sa fixation au récepteur. La poche de liaison au ligand est bordée de résidus hydrophobes prédominant (Figures 4b et 4c). Le ligand étendu englobe l'hélice H3 avec son noyau A (Figures 3b et 3c) orienté vers les extrémités C-terminales de l'hélice H5 et le groupe hydroxyle-25 proche des hélices H7 et H11. Le groupe méthyle 27 forme une faible interaction avec l'hélice H12 (Val 418). Les distances séparant les parties hydroxygle-25 des groupes hydroxyles du noyau A, 1-OH et 3-OH, sont de 13 Å et de 15,4 Å, respectivement.

Dans le complexe, le noyau A adopte une conformation B en chaise avec les groupes 1-OH et 3-OH respectivement en orientations équatoriales et axiales. La partie hydroxyle en position 1 forme deux liaisons H avec Ser 237 (H3) et Arg 274 tandis que le groupe 3-OH forme deux liaisons H avec Ser 278 (H5) et Tyr 143 qui est conservé uniquement chez les mammifères. Arg 274 est compris dans un réseau serré de liaisons H avec des molécules d'eau et le carbonyle de Thr 142 à l'extrémité de H1 (Figure 4c).

Le triène conjugué (Figure 4a), connectant les noyaux A et C est situé dans un canal hydrophobe pris en sandwich entre Ser 275 (boucle H5-β) et Trp 286 (β1) sur l'un des côtés et Leu 233 (H3) sur l'autre côté. La liaison unique C6-C7 présente une conformation trans qui dévie de 30 % par rapport à la géométrie planaire. Cette déviation explique le manque d'activité biologique des analogues ayant une conformation trans ou cis de la liaison C6-C7 (Norman et al., 1997). De façon exclusive, des résidus hydrophobes entourent cette chaîne. Le groupe 25-hydroxyle est lié par liaison H à His 305 (boucle H6-H7) et His 397 (H11) (Figure 4). Un réseau de liaison H autour des résidus histidine indique que His 305 et His 397 sont respectivement les accepteurs et donneurs de liaison H. Tous les résidus impliqués dans le réseau de liaison H à l'exception de Ser 306 sont conservés parmi les VDRs. Les mutants naturels que l'on trouve dans le rachitisme résistant à la vitamine D, Arg274Leu et His305Gln (Kristjansson et al., 1993), confirme le rôle critique pour la liaison au ligand de Arg 274 et His 305, impliqués respectivement dans l'ancrage de 1-OH et 25-OH.

Conformément au fait que le ligand 1,25 (OH)₂D₃ est plus grand que l'estradiol, la progestérone et l'acide rétinoïque tout trans, la poche de liaison au ligand du VDR est plus grande (697 Å³) (Figure 4d) que celle du ER (369 Å³), du PR (427 Å³), du RARγ (421 Å³). Toutefois, l'augmentation de taille n'est pas proportionnelle puisque 1,25 (OH)₂D₃ occupe seulement 56 % de la liaison au ligand du VDR, en comparaison aux 63 %, 67 % et 66 % calculés respectivement avec l'estradiol, la progestérone et l'acide rétinoïque tout trans.

Le volume accessible de la cavité des VDR montre une expansion de la poche à proximité de la position 2 du noyau A, qui est occupée par deux molécules d'eau et représente 40 Å³. Cet espace additionnel pourrait recevoir le groupe méthyle massif du ligand synthétique 2α-méthyl 1,25(OH)₂D₃ qui en effet présente une affinité de liaison 4 fois plus importante que le ligand naturel (Fujishima et al., 1998). De plus, l'espace additionnel autour de la chaîne aliphatique pourrait permettre le logement de différentes longueurs de chaîne.

Plusieurs analogues de la vitamine D ont été montrés comme se comportant différemment du ligand naturel s'agissant de la transactivation et du recrutement de coactivateur (Takeyama et al., 1999 ; Rachez et al., 1998). Afin de comprendre cette spécificité, des études préliminaires du modelage du ligand ont été réalisées. Les ligands synthétiques possédant une chaîne aliphatique plutôt rigide en position 17, tel que MC 903 (22ène-26, 27 -cyclopropyl- 1α, 24S (OH)₂ D₃) ou EB 1089 (22,24 diène - 24, 26, 27 tri-homo 1α, 25 (OH)₂ D₃) (une ou deux double liaisons respectivement), peuvent loger dans la poche de liaison avec seulement quelques ajustements mineurs de la géométrie de 1,25 (OH)₂ D₃. Les noyaux C et D doivent seulement être déplacés afin de loger les groupes méthyles en position 26 et 27 de EB1089 ou le noyau cyclopropyle de MC 903. Pour les analogues 20-epi-1,25 (OH)₂ D₃ et KH 1060, seuls les conformères de faible énergie, avec une conformation gauche-anti autour de C20, peuvent se loger. Avec une telle géométrie, le groupe méthyle en C21 pointe dans la même cavité comme le ligand naturel, tandis que le reste de la chaîne, du à la combinaison de la conformation anti de C20-C22 et de l'épimérisation de C20, borde le côté opposé de la cavité de liaison. Cette variation de chemin conduit à différents contacts pour les deux épimères. La distance de la partie 1-hydroxyle jusqu'à la partie 25-hydroxyle est plus courte dans l'analogue 20-epi, de sorte que les chaînes longues, comme dans le cas de KH1060, peuvent loger. Les ligands avec une chaîne aliphatique plus longue adopte une conformation plus compacte et forment des contacts van der Waals additionnels avec la cavité de liaison qui peut ensuite stabiliser l'hélice en position H12 et/ou affecter des régions moins rigides de la poche de liaison comme la boucle H6-H7.

Ces différents contacts avec une cavité de liaison plutôt rigide peuvent expliquer les différences dans les demi-vies et les activités transcriptionnelles.

La structure de la vitamine D a fait l'objet de nombreuses études ces dix dernières années. La présente invention fournit pour la première fois une image de 1,25 (OH)₂ D₃ dans sa conformation active. Jusqu'à maintenant, la chaîne latérale naturelle de 1,25 (OH)₂ D₃ a été la cible principale des modifications chimiques en vue de découvrir de nouveaux ligands agonistes plus spécifiques.

Le squelette composé des noyaux A à D n'a pas pu être modifié sans perte de la capacité de liaison au ligand. Les analogues auxquels manquent les noyaux entiers C et/ou D mais ayant un espacement normal des groupes hydroxyles peuvent faire des points de contact normaux à l'intérieur de la poche de liaison expliquant leur potentiel biologique normal (Verstuyf et al., 1998).

Le complexe selon l'invention révèle l'arrangement tri-dimensionnel de la poche de liaison autour de 1,25 (OH)₂ D₃ et fournit de nouvelles perspectives pour la conception de squelettes originaux.

### Bibliographie

Bouillon, R., Okamura, W.H. & Norman, A.W. Structure-function relationships in the vitamin D endocrine system. *Endocr. Rev.* **16**, 200-257 (1995)
Bourguet, W., Ruff, M., Chambon, P., Gronemeyer, H. & Moras, D. Crystal structure of the ligand-binding domain of the human nuclear receptor RXR-α. *Nature* **375,** 377-382 (1995)
Brünger, A.T. *et al*., Crystallography & NMR System ; a new software system for macromolecular structure determination. *Acta Cryst*. *D* **54**, 905-921 (1998)
Brzozowski, A.M. et al. Molecular basis of agonism and antagonism in the oestrogen receptor. *Nature* **389**, 753-758 (1997)
Claire, M. *et al.* Statistical test of models and computerised parameter estimation for aldosterone binding in rat kidney. *FEBS Lett.* **88,** 295-299 (1978)
DeLuca H.F. & Zierold C. Mechanisms and functions of vitamin D. *Nutr. Rev.* **56**, 54-75 (1998)
Fujishima, T. *et al.* Synthesis and biological activity of 2-methyl-20-epi analogues of 1,25-dihydroxyvitamin D3. *Bioorg. Med Chem. Lett.* **8**, 2145-2148 (1998)
Jones, T.A., Zou, J.Y., Cowan, S.W. & Kjeldgaard, M. Improved methods for building protein models in electron density maps and the location of errors in these models. *Acta Cryst*. *A* **47**,110-119 (1991)
Klaholz, B.P. *et al*. Conformational adaptation of agonists to the human receptor RARγ. *Nature Struct. Biol.* **5**, 199-202 (1998)
Kristjansson, K., Rut, A.R., Hewison, M., O'Riordan, J.L.H. & Hughes, M.R. Two mutations in the hormone binding domain of vitamin D receptor cause tissue resistance to 1α, 25 - dihydroxyvitamin D₃. *J*. *Clin. Invest.* **92**, 12-16 (1993)
Laskowski, R.A., MacArthur, M.W., Moss, D.S. & Thornton, J.M. PROCHECK : a program to check the stereochemical quality of protein structure coordinates. *J*. *Appl*. *Crystallogr*. **26**, 283-291 (1993)
Mangelsdorf, D.J. *et al*. The nuclear receptor superfamily : the second decade. *Cell* **83**, 835-839 (1995)
Nakajima, S., Yamagata, M., Sakai, N. & Ozono, K. Characterization of the activation function-2 domain of the human 1α, 25 - dihydroxyvitamin D₃ receptor. *Mol*. *Cell*. *Endocr.* **139**, 15-24 (1998)
Navaza, J. Amore : an automated package for molecular replacement. *Acta Cryst. A* **50**, 157-163 (1994)
Norman *et al*. Comparison of 6-s-cis- and 6-s-trans-locked analogs of 1α, 25 - dihydroxyvitamin D₃ indicates that the 6-s-cis conformation is preferred for rapid nongenomic biological responses and that neither 6-s-cis- nor 6-s-trans- locked analogs are preferred for genomic biological responses. *Mol. Endocr.* **11,** 1518-1531 (1997)
Nolte, R.T. et al. Ligand binding and co-activator assembly of the peroxisome proliferator-activated receptor-γ. *Nature* **395,** 137-143 (1998)
Otwinoswski, Z & Minor, W. Processing X-ray data collected in oscillation mode. *Methods in Enzymology* 307-326 (1997)
Rachez, C. et al. A novel protein complex that interacts with vitamin D3 receptor in a ligand-dependant manner and enhances VDR transactivation in a cell-free system. Genes & Dev. 12, 1787-1800 (1998)
Renaud, J.P. *et al..* Crystal structure of the ligand binding domain of the human nuclear receptor RARγ complexed with all-trans retinoic acid. *Nature* **378**, 681-689 (1995)
Suwinska, K. & Kutner, A. Crystal and molecular-structure of 1,25-dihydroxycholecalciferol. *Acta Cryst. B* **52,** 550-554 (1996)
Takeyama, K.I. *et al.* Selective interaction of vitamin D receptor with transcriptional coactivators by a vitamin D analog. *Mol. Cell. Biol.* **19,** 1049-1055 (1999)
Verstuyif, *A. et al.* The biological activity of nonsteroidal vitamin D hormone analogs lacking both the C-rings and D-rings. *J. Bone Miner. Res.* **13**, 549-558 (1998)
Whitfield, G.K., et al. A highly conserved region in the hormone-binding domain of the human vtamin D receptor contains residues vital for heterodimerization with retinoid X receptor and for transcriptional activation. Mol. Endocr. 9, 1166-1179 (1995)
Wurtz, J.M. *et al.* A canonical structure for the ligand-binding domain of nuclear receptors. *Nature Struct. Biol.* **3**, 87-94 (1996)
Xiao, J.H., Davidson, I., Matthes, H., Garnier, J.M. & Chambon, P. Cloning expression and transcriptional properties of the human enhancer factor TEF-1. *Cell* **65,** 551-568 (1991)

## Revendications

1. Polypeptides dérivés du récepteur nucléaire de la vitamine D chez l'homme ou les différentes espèces du règne animal possédant un tel récepteur, ledit récepteur nucléaire comprenant un domaine de liaison au ligand, ou LBD, ce LBD contenant un domaine d'insertion flexible, lesdits polypeptides dérivés étant **caractérisés en ce qu'**ils comprennent les séquences peptidiques desdits récepteurs nucléaires dans lesquelles est supprimé le fragment peptidique délimité :
• d'une part, par un acide aminé situé approximativement à l'une des positions 155 à 175 des séquences peptidiques des récepteurs nucléaires de la vitamine D d'origine humaine ou animale, et plus particulièrement par l'acide aminé situé à l'une des positions 159 à 168 de ces séquences,
• et, d'autre part, par un acide aminé situé approximativement à l'une des positions 204 à 225 des séquences peptidiques des récepteurs nucléaires de la vitamine D d'origine humaine ou animale, lesdits polypeptides dérivés possédant les caractéristiques suivantes :
* les propriétés de liaison au ligand et de transactivation du LBD du récepteur de la vitamine D sont conservées,
* ils sont stables, à savoir qu'ils peuvent être conservés, notamment dans du NaCl 100 mM à pH 7 pendant au moins environ une semaine, sans que soit pour autant affectées les propriétés susmentionnées du LBD, par opposition au LBD non modifié qui est instable dans les conditions susmentionnées,
* ils sont cristallisables dans des solvants aqueux, notamment à 4°C par la méthode de diffusion de vapeur à goutte suspendue,
* et, ils sont solubles dans des solvants aqueux,

2. Polypeptides dérivés selon la revendication 1, lesdits polypeptides dérivés étant choisis parmi ceux comprenant la séquence en acides aminés délimitée par les acides aminés situés aux positions 118 et 427, ou aux positions 124 et 427 des séquences peptidiques des VDR d'origine humaine ou animale, et dans laquelle les résidus situés aux positions 165 à 215 desdites séquences peptidiques des VDR sont supprimés.

3. Polypeptides dérivés selon l'une des revendications 1 ou 2, choisis parmi les polypeptides SEQ ID NO : 4, SEQ ID NO : 6, et SEQ ID NO : 8.

4. Cristaux comprenant un polypeptide selon l'une des revendications 1 à 3, le cas échéant en association avec la vitamine D, ou avec un analogue agoniste ou antagoniste de la vitamine D.

5. Séquence nucléotidique codant pour un polypeptide selon l'une des revendications 1 à 3.

6. Séquence nucléotidique selon la revendication 5, choisie parmi :
- les séquences SEQ ID NO : 3, SEQ ID NO : 5 et SEQ ID NO : 7,
- ou une séquence nucléotidique dérivée par dégénérescence du code génétique des séquences nucléotidiques susmentionnées, et codant pour un polypeptide dérivé du hVDR selon la revendication 3.

7. Séquence nucléotidique recombinante comprenant une séquence nucléotidique selon la revendication 5 ou 6 en association avec les éléments nécessaires à la transcription de cette séquence, notamment avec un promoteur et un terminateur de transcription.

8. Vecteur, notamment plasmide, contenant une séquence nucléotidique selon l'une des revendications 5 à 7.

9. Cellules hôtes transformées par un vecteur selon la revendication 8, lesdites cellules étant choisies notamment parmi les bactéries telles que *E. coli,* ou les cellules d'insectes susceptibles d'être infectées par un baculovirus.

10. Procédé de préparation d'un polypeptide selon l'une des revendications 1 à 3, **caractérisé en ce qu'**il comprend les étapes suivantes :
- transformation de cellules à l'aide d'un vecteur recombinant selon la revendication 8,
- mise en culture des cellules ainsi transformées, et récupération dudit polypeptide produit par lesdites cellules, le cas échéant après purification.

11. Utilisation d'un polypeptide selon l'une des revendications 1 à 3, ou de cristaux selon la revendication 4, pour la mise en oeuvre d'une méthode de criblage d'analogues de la vitamine D.

12. Utilisation selon la revendication 11, pour la mise en oeuvre d'une méthode de criblage d'analogues agonistes ou antagonistes de la vitamine D susceptibles d'être utilisés dans des compositions pharmaceutiques, notamment dans le cadre du traitement de pathologies cancéreuses, du psoriasis, des maladies auto-immunes, de l'ostéodystrophie ou de l'ostéoporose.

13. Méthode de criblage d'analogues de la vitamine D, **caractérisée en ce qu'**elle comprend les étapes suivantes :
- mise en présence d'un polypeptide selon l'une des revendications 1 à 3, ou de cristaux selon la revendication 4, avantageusement lié à un support solide, avec l'analogue testé, l'un dudit polypeptide ou de l'analogue de la vitamine D étant avantageusement marqué, notamment à l'aide d'un marqueur fluorescent, radioactif ou enzymatique,
- détection de l'éventuelle liaison entre ledit polypeptide, ou lesdits cristaux, et l'analogue testé par mesure du marqueur utilisé, notamment après rinçage du support utilisé lors de l'étape précédente.

14. Utilisation d'un polypeptide selon l'une des revendications 1 à 3, ou de cristaux selon la revendication 4, pour la mise en oeuvre d'une méthode d'analyse de la structure tridimensionnelle des complexes formés entre ledit polypeptide, ou lesdits cristaux, et une molécule déterminée.

15. Méthode d'analyse de la structure tridimensionnelle des complexes formés entre un polypeptide selon l'une des revendications 1 à 3, ou de cristaux selon la revendication 4, et une molécule déterminée, ledit procédé comprenant les étapes suivantes :
- mise en présence dudit polypeptide dérivé, ou desdits cristaux, avec la molécule déterminée,
- cristallisation du complexe formé entre ledit polypeptide dérivé, ou lesdits cristaux, et la molécule déterminée, notamment par diffusion de vapeur, et analyse tridimensionnelle dudit complexe notamment par remplacement moléculaire,
- ou analyse tridimensionnelle dudit complexe en phase soluble, notamment par RMN.

## Claims

1. Polypeptides derived from vitamin D nuclear receptor in humans or different animal species possessing such a receptor, said nuclear receptor comprising a ligand-binding domain, or LBD, this LBD containing a flexible insertion domain, said derived polypeptides being **characterised in that** they comprise the peptide sequences of said nuclear receptors in which a peptide fragment is suppressed, said peptide fragment being delimited:
- on the one hand, by an amino acid situated approximately at one of the positions 155 to 175 of the peptide sequences of vitamin D nuclear receptors of human or animal origin, and more particularly by the amino acid situated at one of the positions 159 to 168 of these sequences,
- and, on the other hand, by an amino acid situated approximately at one of the positions 204 to 225 of the peptide sequences of vitamin D nuclear receptors of human or animal origin,
said derived polypeptides having the following characteristics:
* the ligand-binding and LBD-transactivation properties of the vitamin D receptor are conserved,
* they are stable, i.e. they can be conserved, in particular in NaCl 100 mM at pH 7 for at least approximately one week, without the above-mentioned properties of the LBD being affected, in contrast to the non-modified LBD which is unstable under the above-mentioned conditions.
* they can be crystallised in aqueous solvents, in particular at 4°C by the suspended droplet vapour diffusion method,
* and they are soluble in aqueous solvents,

2. Polypeptides derived according to claim 1, said derived polypeptides being chosen from those comprising the amino acid sequence delimited by the amino acids situated at positions 118 and 427, or at positions 124 and 427 of the peptide sequences of VDRs of human or animal origin, and in which the residues situated at positions 165 to 215 of said peptide sequences of the VDRs are suppressed.

3. Polypeptides derived according to any one of claims 1 or 2, chosen from the polypeptides SEQ ID NO : 4, SEQ ID NO : 6, and SEQ ID NO : 8.

4. Crystals comprising a polypeptide according to any one of claims 1 to 3, optionally in association with vitamin D, or an agonistic or antagonistic vitamin D analogue.

5. Nucleotide sequence coding for a polypeptide according to any one of claims 1 to 3.

6. Nucleotide sequence according to claim 5, chosen from:
- the sequences SEQ ID NO : 3, SEQ ID NO : 5, and SEQ ID NO : 7,
- or a nucleotide sequence derived by degenerescence of the genetic code of the above-mentioned nucleotide sequences, and coding for a polypeptide derived from the hVDR according to claim 3.

7. Recombinant nucleotide sequence comprising a nucleotide sequence according to claim 5 or 6, in association with the elements necessary for transcription of this sequence, in particular with a promoter and a transcription terminator.

8. Vector, in particular plasmid, containing a nucleotide sequence according to any one of claims 5 to 7.

9. Host cells transformed by a vector according to claim 8, said cells being chosen in particular from bacteria such as *E. coli*, or insect cells which can be infected by a baculovirus.

10. Process for the preparation of a polypeptide according to any one of claims 1 to 3, **characterised in that** it comprises the following steps:
- transformation of cells by means of a recombinant vector according to claim 8,
- culturing of the cells thus transformed and recovery of said polypeptide produced by said cells, optionally after purification.

11. Use of a polypeptide according to any one of claims 1 to 3, or of crystals according to claim 4 for the implementation of a method for screening vitamin D analogues.

12. Use according to claim 11, for the implementation of a method for screening agonistic or antagonistic vitamin D analogues which can be used in pharmaceutical compositions, in particular in the treatment of cancerous pathologies, psoriasis, autoimmune diseases, osteodystrophy, or osteoporosis.

13. Method for screening vitamin D analogues, **characterised in that** it comprises the following steps:
- introduction of a polypeptide according to any one of claims 1 to 3, or of crystals according to claim 4, advantageously bound to a solid support, with the analogue tested, one of said derived polypeptides or vitamin D analogues being advantageously marked, in particular by means of a fluorescent, radioactive or enzymatic marker,
- detection of any bond between said polypeptide, or said crystals and tested analogue by measuring the marker used, in particular after rinsing of the support used during the previous step.

14. Use of a polypeptide according to any one of claims 1 to 3, or of crystals according to claim 4, for implementation of a method of analysis of the three-dimensional structure of the complexes formed between said polypeptide, or said crystals, and a specific molecule.

15. Method for analysis of the three-dimensional structure of the complexes formed between a polypeptide according to any one of claims 1 to 3, or of crystals according to claim 4, and a specific molecule, said process comprising the following steps:
- introduction of said derived polypeptide, or said crystals, with the specific molecule,
- crystallisation of the complex formed between said derived polypeptide, or said crystals, and the specific molecule, in particular by vapour diffusion, and three-dimensional analysis of said complex, in particular by molecular replacement,
- or three-dimensional analysis of said complex in soluble phase, in particular by NMR.

## Patentansprüche

1. Polypeptide, die vom Vitamin-D-Kernrezeptor beim Menschen oder verschiedenen Spezies des Tierreichs, die einen solchen Rezeptor besitzen, abgeleitet sind, wobei der Kernrezeptor eine Ligandenbindungsdomäne, oder LBD, enthält, diese LBD eine flexible Insertionsdomäne enthält, die abgeleiteten Polypeptide **dadurch gekennzeichnet sind, dass** sie die Peptidsequenzen der Kernrezeptoren enthalten, worin das Peptidfragment unterdrückt ist, das begrenzt ist:
• einerseits durch eine Aminosäure, die ungefähr an einer der Positionen 155 bis 175 der Peptidsequenzen der Vitamin-D-Kernrezeptoren menschlichen oder tierischen Ursprungs angeordnet ist, und insbesondere durch die Aminosäure, die an einer der Positionen 159 bis 168 dieser Sequenzen angeordnet ist,
• und andererseits durch eine Aminosäure, die ungefähr an einer der Positionen 204 bis 225 der Peptidsequenzen der Vitamin-D-Kernrezeptoren menschlichen oder tierischen Ursprungs angeordnet ist,
wobei die abgeleiteten Polypeptide folgende Eigenschaften besitzen:
* die Eigenschaften der Ligandenbindung und der Transaktivierung der LBD des Vitamin-D-Rezeptors bleiben erhalten,
* sie sind stabil, und zwar können sie insbesondere in 100 mM NaCl bei pH 7 mindestens ungefähr eine Woche konserviert werden, ohne dass die oben genannten Eigenschaften der LBD berührt werden, im Gegensatz zur nicht modifizierten LBD, die unter den oben genannten Bedingungen instabil ist,
* sie sind in wässrigen Lösungsmitteln kristallisierbar, insbesondere bei 4 °C durch die Methode der Dampfdiffusion im hängenden Tropfen,
* und sie sind in wässrigen Lösungsmitteln löslich.

2. Abgeleitete Polypeptide gemäß Anspruch 1, wobei diese abgeleiteten Polypeptide aus jenen ausgewählt sind, die die Aminosäuresequenz enthalten, die durch die Aminosäuren, die an den Positionen 118 und 427 oder an den Positionen 124 und 427 der Peptidsequenzen der VDR menschlichen oder tierischen Ursprungs gelegen sind, begrenzt ist und worin die Reste, die an den Positionen 165 bis 215 der Peptidsequenzen der VDR angeordnet sind, unterdrückt sind.

3. Abgeleitete Polypeptide gemäß einem der Ansprüche 1 oder 2, ausgewählt aus den Polypeptiden SEQ ID NO. 4, SEQ ID NO. 6 und SEQ ID NO. 8.

4. Kristalle, die ein Polypeptid gemäß einem der Ansprüche 1 bis 3 enthalten, gegebenenfalls in Verbindung mit Vitamin D oder mit einem agonistischen oder antagonistischen Analogon von Vitamin D.

5. Nucleotidsequenz, die für ein Polypeptid gemäß einem der Ansprüche 1 bis 3 codiert.

6. Nucleotidsequenz gemäß Anspruch 5, ausgewählt aus:
- den Sequenzen SEQ ID NO. 3, SEQ ID NO. 5 und SEQ ID NO. 7,
- oder einer Nucleotidsequenz, die durch Degenerierung des genetischen Codes von den oben genannten Nucleotidsequenzen abgeleitet ist und für ein Polypeptid codiert, das vom hVDR abgeleitet ist, gemäß Anspruch 3.

7. Rekombinante Nucleotidsequenz, umfassend eine Nucleotidsequenz gemäß Anspruch 5 oder 6 in Verbindung mit den für die Transkription dieser Sequenz notwendigen Elementen, insbesondere mit einem Promotor und einem Transkriptionsterminator.

8. Vektor, insbesondere Plasmid, enthaltend eine Nucleotidsequenz gemäß einem der Ansprüche 5 bis 7.

9. Wirtszellen, transformiert durch einen Vektor gemäß Anspruch 8, wobei die Zellen insbesondere aus Bakterien wie *E. coli* oder Insektenzellen, die durch ein Baculovirus infizierbar sind, ausgewählt sind.

10. Verfahren zur Herstellung eines Polypeptids gemäß einem der Ansprüche 1 bis 3, **dadurch gekennzeichnet, dass** es die folgenden Schritte umfasst:
- Transformation von Zellen mit Hilfe eines rekombinanten Vektors gemäß Anspruch 8,
- Inkulturbringen der so transformierten Zellen und Gewinnung des durch diese Zellen produzierten Polypeptids, gegebenenfalls nach Reinigung.

11. Verwendung eines Polypeptids gemäß einem der Ansprüche 1 bis 3 oder von Kristallen gemäß Anspruch 4 zur Durchführung eines Screening-Verfahrens für Vitamin-D-Analoga.

12. Verwendung gemäß Anspruch 11 zum Durchführen eines Screening-Verfahrens für agonistische oder antagonistische Analoga von Vitamin D, die in pharmazeutischen Zusammensetzungen verwendet werden können, insbesondere im Rahmen der Behandlung von Krebserkrankungen, Psoriasis, Autoimmunerkrankungen, Osteodystrophie oder Osteoporose.

13. Screening-Verfahren für Analoga von Vitamin D, **dadurch gekennzeichnet, dass** es folgende Schritte umfasst:
- Inkontaktbringen eines Polypeptids gemäß einem der Ansprüche 1 bis 3 oder von Kristallen gemäß Anspruch 4, vorzugsweise an einen festen Träger gebunden, mit dem Test-Analogon, wobei vorzugsweise entweder das Polypeptid oder das Vitamin-D-Analogon markiert ist, insbesondere mit Hilfe eines fluoreszierenden, radioaktiven oder enzymatischen Markers,
- Detektieren der gegebenenfalls vorhandenen Bindung zwischen dem Polypeptid oder den Kristallen und dem Test-Analogon durch Messen des verwendeten Markers, insbesondere nach Spülen des im vorangegangenen Schritt verwendeten Trägers.

14. Verwendung eines Polypeptids gemäß einem der Ansprüche 1 bis 3 oder von Kristallen gemäß Anspruch 4 zur Durchführung eines Verfahrens zur Analyse der dreidimensionalen Struktur von Komplexen, die zwischen dem Polypeptid oder den Kristallen und einem bestimmten Molekül gebildet sind.

15. Verfahren zur Analyse der dreidimensionalen Struktur von Komplexen, die zwischen einem Polypeptid gemäß einem der Ansprüche 1 bis 3 oder Kristallen gemäß Anspruch 4 und einem bestimmten Molekül gebildet sind, wobei das Verfahren die folgenden Schritte umfasst:
- Inkontaktbringen des abgeleiteten Polypeptids oder der Kristalle mit dem bestimmten Molekül,
- Kristallisieren des zwischen dem abgeleiteten Polypeptid oder den Kristallen und dem bestimmten Molekül gebildeten Komplexes, insbesondere durch Dampfdiffusion, und dreidimensionale Analyse des Komplexes, insbesondere durch Molecular Replacement,
- oder dreidimensionale Analyse des Komplexes in löslicher Phase, insbesondere durch NMR.


### LISTE DE SEQUENCES

<110> CNRS
<120> POLYPEPTIDES DERIVES DU RECEPTEUR NUCLEAIRE DE LA VITAMINE D, ET LEURS UTILISATIONS NOTAMMENT DANS LE CADRE DU CRIBLAGE D'ANALOGUES DE LA VITAMINE D
<130> WOB 99 CNR VID3
<140>
   <141>
<150> FR9914633
   <151> 1999-11-22
<160> 8
<170> PatentIn Ver. 2.1
<210> 1
   <211> 1284
   <212> ADN
   <213> Homo sapiens
<220>
   <221> CDS
   <222> (1)..(1281)
<400> 1
atg gag gca atg gcg gcc agc act tcc ctg cct gac cct gga gac ttt      48
   gac cgg aac gtg ccc cgg atc tgt ggg gtg tgt gga gac cga gcc act      96
   ggc ttt cac ttc aat gct atg acc tgt gaa ggc tgc aaa ggc ttc ttc     144
   agg cga agc atg aag cgg aag gca cta ttc acc tgc ccc ttc aac ggg     192
   gac tgc cgc atc acc aag gac aac cga cgc cac tgc cag gcc tgc cgg     240
   ctc aaa cgc tgt gtg gac atc ggc atg atg aag gag ttc att ctg aca     288
   gat gag gaa gtg cag agg aag cgg gag atg atc ctg aag cgg aag gag     336
   gag gag gcc ttg aag gac agt ctg cgg ccc aag ctg tct gag gag cag     384
   cag cgc atc att gcc ata ctg ctg gac gcc cac cat aag acc tac gac 432
   ccc acc tac tcc gac ttc tgc cag ttc cgg cct cca gtt cgt gtg aat 480
   gat ggt gga ggg agc cat cct tcc agg ccc aac tcc aga cac act ccc 528
   agc ttc tct ggg gac tcc tcc tcc tcc tgc tca gat cac tgt atc acc    576
   tct tca gac atg atg gac tcg tcc agc ttc tcc aat ctg gat ctg agt 624
   gaa gaa gat tca gat gac cct tct gtg acc cta gag ctg tcc cag ctc 672
   tcc atg ctg ccc cac ctg gct gac ctg gtc agt tac agc atc caa aag 720
   gtc att ggc ttt gct aag atg ata cca gga ttc aga gac ctc acc tct 768
   gag gac cag atc gta ctg ctg aag tca agt gcc att gag gtc atc atg 816
   ttg cgc tcc aat gag tcc ttc acc atg gac gac atg tcc tgg acc tgt 864
   ggc aac caa gac tac aag tac cgc gtc agt gac gtg acc aaa gcc gga 912
   cac agc ctg gag ctg att gag ccc ctc atc aag ttc cag gtg gga ctg 960
   aag aag ctg aac ttg cat gag gag gag cat gtc ctg ctc atg gcc atc 1008
   tgc atc gtc tcc cca gat cgt cct ggg gtg cag gac gcc gcg ctg atc 1056
   gag gcc atc cag gac cgc ctg tcc aac aca ctg cag acg tac atc cgc 1104
   tgc cgc cac ccg ccc ccg ggc agc cac ctg ctc tat gcc aag atg atc      1152
   cag aag cta gcc gac ctg cgc agc ctc aat gag gag cac tcc aag cag      1200
   tac cgc tgc ctc tcc ttc cag cct gag tgc agc atg aag cta acg ccc      1248
   ctt gtg ctc gaa gtg ttt ggc aat gag atc tcc tga                      1284
<210> 2
   <211> 427
   <212> PRT
   <213> Homo sapiens
<400> 2
<210> 3
   <211> 1131
   <212> ADN
   <213> Séquence artificielle
<220>
   <223> Description de la séquence artificielle: séquence nucléotidique codant un polypeptide dérivé du récepteur nucléaire de la vitamine D humain
<220>
   <221> CDS
   <222> (1)..(1128)
<400> 3
   atg gag gca atg gcg gcc agc act tcc ctg cct gac cct gga gac ttt    48
   Met Glu Ala Met Ala Ala Ser Thr Ser Leu Pro Asp Pro Gly Asp Phe
    1               5                  10                  15
   gac cgg aac gtg ccc cgg atc tgt ggg gtg tgt gga gac cga gcc act    96
   Asp Arg Asn Val Pro Arg Ile Cys Gly Val Cys Gly Asp Arg Ala Thr
               20                  25                  30
   ggc ttt cac ttc aat gct atg acc tgt gaa ggc tgc aaa ggc ttc ttc   144
   Gly Phe His Phe Asn Ala Met Thr Cys Glu Gly Cys Lys Gly Phe Phe
           35                  40                  45
   agg cga agc atg aag cgg aag gca cta ttc acc tgc ccc ttc aac ggg   192
   Arg Arg Ser Met Lys Arg Lys Ala Leu Phe Thr Cys Pro Phe Asn Gly
       50                  55                  60
   gac tgc cgc atc acc aag gac aac cga cgc cac tgc cag gcc tgc cgg   240
   Asp Cys Arg Ile Thr Lys Asp Asn Arg Arg His Cys Gln Ala Cys Arg
   65                  70                  75                  80
   ctc aaa cgc tgt gtg gac atc ggc atg atg aag gag ttc att ctg aca   288
   Leu Lys Arg Cys Val Asp Ile Gly Met Met Lys Glu Phe Ile Leu Thr
                   85                  90                  95
   gat gag gaa gtg cag agg aag cgg gag atg atc ctg aag cgg aag gag   336
   Asp Glu Glu Val Gln Arg Lys Arg Glu Met Ile Leu Lys Arg Lys Glu
               100                 105                 110
   gag gag gcc ttg aag gac agt ctg cgg ccc aag ctg tct gag gag cag   384
   Glu Glu Ala Leu Lys Asp Ser Leu Arg Pro Lys Leu Ser Glu Glu Gln
           115                 120                 125
   cag cgc atc att gcc ata ctg ctg gac gcc cac cat aag acc tac gac   432
   Gln Arg Ile Ile Ala Ile Leu Leu Asp Ala His His Lys Thr Tyr Asp
       130                 135                 140
   ccc acc tac tcc gac ttc tgc cag ttc cgg cct cca gtt cgt gtg aat   480
   Pro Thr Tyr Ser Asp Phe Cys Gln Phe Arg Pro Pro Val Arg Val Asn
   145                 150                 155                 160
   gat ggt gga ggg agc gtg acc cta gag ctg tcc cag ctc tcc atg ctg   528
   Asp Gly Gly Gly Ser Val Thr Leu Glu Leu Ser Gln Leu Ser Met Leu
                   165                 170                 175
   ccc cac ctg gct gac ctg gtc agt tac agc atc caa aag gtc att ggc   576
   Pro His Leu Ala Asp Leu Val Ser Tyr Ser Ile Gln Lys Val Ile Gly
               180                 185                 190
   ttt gct aag atg ata cca gga ttc aga gac ctc acc tct gag gac cag   624
   Phe Ala Lys Met Ile Pro Gly Phe Arg Asp Leu Thr Ser Glu Asp Gln
           195                 200                 205
   atc gta ctg ctg aag tca agt gcc att gag gtc atc atg ttg cgc tcc   672
   Ile Val Leu Leu Lys Ser Ser Ala Ile Glu Val Ile Met Leu Arg Ser
       210                 215                 220
   aat gag tcc ttc acc atg gac gac atg tcc tgg acc tgt ggc aac caa   720
   Asn Glu Ser Phe Thr Met Asp Asp Met Ser Trp Thr Cys Gly Asn Gln
   225                 230                 235                 240
   gac tac aag tac cgc gtc agt gac gtg acc aaa gcc gga cac agc ctg    768
   Asp Tyr Lys Tyr Arg Val Ser Asp Val Thr Lys Ala Gly His Ser Leu
                   245                 250                 255
   gag ctg att gag ccc ctc atc aag ttc cag gtg gga ctg aag aag ctg    816
   Glu Leu Ile Glu Pro Leu Ile Lys Phe Gln Val Gly Leu Lys Lys Leu
               260                 265                 270
   aac ttg cat gag gag gag cat gtc ctg ctc atg gcc atc tgc atc gtc    864
   Asn Leu His Glu Glu Glu His Val Leu Leu Met Ala Ile Cys Ile Val
           275                 280                 285
   tcc cca gat cgt cct ggg gtg cag gac gcc gcg ctg atc gag gcc atc    912
   Ser Pro Asp Arg Pro Gly Val Gln Asp Ala Ala Leu Ile Glu Ala Ile
       290                 295                 300
   cag gac cgc ctg tcc aac aca ctg cag acg tac atc cgc tgc cgc cac    960
   Gln Asp Arg Leu Ser Asn Thr Leu Gln Thr Tyr Ile Arg Cys Arg His
   305                 310                 315                 320
   ccg ccc ccg ggc agc cac ctg ctc tat gcc aag atg atc cag aag cta    1008
   Pro Pro Pro Gly Ser His Leu Leu Tyr Ala Lys Met Ile Gln Lys Leu
                   325                 330                 335
   gcc gac ctg cgc agc ctc aat gag gag cac tcc aag cag tac cgc tgc    1056
   Ala Asp Leu Arg Ser Leu Asn Glu Glu His Ser Lys Gln Tyr Arg Cys
               340                 345                 350
   ctc tcc ttc cag cct gag tgc agc atg aag cta acg ccc ctt gtg ctc    1104
   Leu Ser Phe Gln Pro Glu Cys Ser Met Lys Leu Thr Pro Leu Val Leu
           355                 360                 365
   gaa gtg ttt ggc aat gag atc tcc tga                                1131
   Glu Val Phe Gly Asn Glu Ile Ser
       370                 375
<210> 4
   <211> 376
   <212> PRT
   <213> Séquence artificielle
   <223> Description de la séquence artificielle: séquence nucléotidique codant un polypeptide dérivé du récepteur nucléaire de la vitamine D humain
<400> 4
   Met Glu Ala Met Ala Ala Ser Thr Ser Leu Pro Asp Pro Gly Asp Phe
    1               5                  10                  15
   Asp Arg Asn Val Pro Arg Ile Cys Gly Val Cys Gly Asp Arg Ala Thr
               20                  25                  30
   Gly Phe His Phe Asn Ala Met Thr Cys Glu Gly Cys Lys Gly Phe Phe
           35                  40                  45
   Arg Arg Ser Met Lys Arg Lys Ala Leu Phe Thr Cys Pro Phe Asn Gly
       50                  55                  60
   Asp Cys Arg Ile Thr Lys Asp Asn Arg Arg His Cys Gln Ala Cys Arg
   65                  70                  75                  80
   Leu Lys Arg Cys Val Asp Ile Gly Met Met Lys Glu Phe Ile Leu Thr
                   85                  90                  95
   Asp Glu Glu Val Gln Arg Lys Arg Glu Met Ile Leu Lys Arg Lys Glu
               100                 105                 110
   Glu Glu Ala Leu Lys Asp Ser Leu Arg Pro Lys Leu Ser Glu Glu Gln
           115                 120                 125
   Gln Arg Ile Ile Ala Ile Leu Leu Asp Ala His His Lys Thr Tyr Asp
       130                 135                 140
   Pro Thr Tyr Ser Asp Phe Cys Gln Phe Arg Pro Pro Val Arg Val Asn
   145                 150                 155                 160
   Asp Gly Gly Gly Ser Val Thr Leu Glu Leu Ser Gln Leu Ser Met Leu
                   165                 170                 175
   Pro His Leu Ala Asp Leu Val Ser Tyr Ser Ile Gln Lys Val Ile Gly
               180                 185                 190
   Phe Ala Lys Met Ile Pro Gly Phe Arg Asp Leu Thr Ser Glu Asp Gln
           195                 200                 205
   Ile Val Leu Leu Lys Ser Ser Ala Ile Glu Val Ile Met Leu Arg Ser
       210                 215                 220
   Asn Glu Ser Phe Thr Met Asp Asp Met Ser Trp Thr Cys Gly Asn Gln
   225                 230                 235                 240
   Asp Tyr Lys Tyr Arg Val Ser Asp Val Thr Lys Ala Gly His Ser Leu
                   245                 250                 255
   Glu Leu Ile Glu Pro Leu Ile Lys Phe Gln Val Gly Leu Lys Lys Leu
               260                 265                 270
   Asn Leu His Glu Glu Glu His Val Leu Leu Met Ala Ile Cys Ile Val
           275                 280                 285
   Ser Pro Asp Arg Pro Gly Val Gln Asp Ala Ala Leu Ile Glu Ala Ile
       290                 295                 300
   Gln Asp Arg Leu Ser Asn Thr Leu Gln Thr Tyr Ile Arg Cys Arg His
   305                 310                 315                 320
   Pro Pro Pro Gly Ser His Leu Leu Tyr Ala Lys Met Ile Gln Lys Leu
                   325                 330                 335
   Ala Asp Leu Arg Ser Leu Asn Glu Glu His Ser Lys Gln Tyr Arg Cys
               340                 345                 350
   Leu Ser Phe Gln Pro Glu Cys Ser Met Lys Leu Thr Pro Leu Val Leu
           355                 360                 365
   Glu Val Phe Gly Asn Glu Ile Ser
       370                 375
<210> 5
   <211> 780
   <212> ADN
   <213> Séquence artificielle
<220>
   <223> Description de la séquence artificielle: séquence nucléotidique codant un polypeptide dérivé du récepteur nucléaire de la vitamine D humain
<220>
   <221> CDS
   <222> (1)..(777)
<400> 5
   gac agt ctg cgg ccc aag ctg tct gag gag cag cag cgc atc att gcc      48
   Asp Ser Leu Arg Pro Lys Leu Ser Glu Glu Gln Gln Arg Ile Ile Ala
    1               5                  10                  15
   ata ctg ctg gac gcc cac cat aag acc tac gac ccc acc tac tcc gac      96
   Ile Leu Leu Asp Ala His His Lys Thr Tyr Asp Pro Thr Tyr Ser Asp
               20                  25                  30
   ttc tgc cag ttc cgg cct cca gtt cgt gtg aat gat ggt gga ggg tct     144
   Phe Cys Gln Phe Arg Pro Pro Val Arg Val Asn Asp Gly Gly Gly Ser
           35                  40                  45
   gtg acc cta gag ctg tcc cag ctc tcc atg ctg ccc cac ctg gct gac     192
   Val Thr Leu Glu Leu Ser Gln Leu Ser Met Leu Pro His Leu Ala Asp
       50                  55                  60
   ctg gtc agt tac agc atc caa aag gtc att ggc ttt gct aag atg ata     240
   Leu Val Ser Tyr Ser Ile Gln Lys Val Ile Gly Phe Ala Lys Met Ile
   65                  70                  75                  80
   cca gga ttc aga gac ctc acc tct gag gac cag atc gta ctg ctg aag     288
   Pro Gly Phe Arg Asp Leu Thr Ser Glu Asp Gln Ile Val Leu Leu Lys
                   85                  90                  95
   tca agt gcc att gag gtc atc atg ttg cgc tcc aat gag tcc ttc acc     336
   Ser Ser Ala Ile Glu Val Ile Met Leu Arg Ser Asn Glu Ser Phe Thr
               100                 105                 110
   atg gac gac atg tcc tgg acc tgt ggc aac caa gac tac aag tac cgc     384
   Met Asp Asp Met Ser Trp Thr Cys Gly Asn Gln Asp Tyr Lys Tyr Arg
           115                 120                 125
   gtc agt gac gtg acc aaa gcc gga cac agc ctg gag ctg att gag ccc     432
   Val Ser Asp Val Thr Lys Ala Gly His Ser Leu Glu Leu Ile Glu Pro
       130                 135                 140
   ctc atc aag ttc cag gtg gga ctg aag aag ctg aac ttg cat gag gag     480
   Leu Ile Lys Phe Gln Val Gly Leu Lys Lys Leu Asn Leu His Glu Glu
   145                 150                 155                 160
   gag cat gtc ctg ctc atg gcc atc tgc atc gtc tcc cca gat cgt cct     528
   Glu His Val Leu Leu Met Ala Ile Cys Ile Val Ser Pro Asp Arg Pro
                   165                 170                 175
   ggg gtg cag gac gcc gcg ctg atc gag gcc atc cag gac cgc ctg tcc     576
   Gly Val Gln Asp Ala Ala Leu Ile Glu Ala Ile Gln Asp Arg Leu Ser
               180                 185                 190
   aac aca ctg cag acg tac atc cgc tgc cgc cac ccg ccc ccg ggc agc    624
   Asn Thr Leu Gln Thr Tyr Ile Arg Cys Arg His Pro Pro Pro Gly Ser
           195                 200                 205
   cac ctg ctc tat gcc aag atg atc cag aag cta gcc gac ctg cgc agc    672
   His Leu Leu Tyr Ala Lys Met Ile Gln Lys Leu Ala Asp Leu Arg Ser
       210                 215                 220
   ctc aat gag gag cac tcc aag cag tac cgc tgc ctc tcc ttc cag cct    720
   Leu Asn Glu Glu His Ser Lys Gln Tyr Arg Cys Leu Ser Phe Gln Pro
   225                 230                 235                 240
   gag tgc agc atg aag cta acg ccc ctt gtg ctc gaa gtg ttt ggc aat    768
   Glu Cys Ser Met Lys Leu Thr Pro Leu Val Leu Glu Val Phe Gly Asn
                   245                 250                 255
   gag atc tcc tga                                                    780
   Glu Ile Ser
<210> 6
   <211> 259
   <212> PRT
   <213> Séquence artificielle
   <223> Description de la séquence artificielle: séquence nucléotidique codant un polypeptide dérivé du récepteur nucléaire de la vitamine D humain
<400> 6
   Asp Ser Leu Arg Pro Lys Leu Ser Glu Glu Gln Gln Arg Ile Ile Ala
    1               5                  10                  15
   Ile Leu Leu Asp Ala His His Lys Thr Tyr Asp Pro Thr Tyr Ser Asp
               20                  25                  30
   Phe Cys Gln Phe Arg Pro Pro Val Arg Val Asn Asp Gly Gly Gly Ser
           35                  40                  45
   Val Thr Leu Glu Leu Ser Gln Leu Ser Met Leu Pro His Leu Ala Asp
       50                  55                  60
   Leu Val Ser Tyr Ser Ile Gln Lys Val Ile Gly Phe Ala Lys Met Ile
   65                  70                  75                  80
   Pro Gly Phe Arg Asp Leu Thr Ser Glu Asp Gln Ile Val Leu Leu Lys
                   85                  90                  95
   Ser Ser Ala Ile Glu Val Ile Met Leu Arg Ser Asn Glu Ser Phe Thr
               100                 105                 110
   Met Asp Asp Met Ser Trp Thr Cys Gly Asn Gln Asp Tyr Lys Tyr Arg
           115                 120                 125
   Val Ser Asp Val Thr Lys Ala Gly His Ser Leu Glu Leu Ile Glu Pro
       130                 135                 140
   Leu Ile Lys Phe Gln Val Gly Leu Lys Lys Leu Asn Leu His Glu Glu
   145                 150                 155                 160
   Glu His Val Leu Leu Met Ala Ile Cys Ile Val Ser Pro Asp Arg Pro
                   165                 170                 175
   Gly Val Gln Asp Ala Ala Leu Ile Glu Ala Ile Gln Asp Arg Leu Ser
               180                 185                 190
   Asn Thr Leu Gln Thr Tyr Ile Arg Cys Arg His Pro Pro Pro Gly Ser
           195                 200                 205
   His Leu Leu Tyr Ala Lys Met Ile Gln Lys Leu Ala Asp Leu Arg Ser
       210                 215                 220
   Leu Asn Glu Glu His Ser Lys Gln Tyr Arg Cys Leu Ser Phe Gln Pro
   225                 230                 235                 240
   Glu Cys Ser Met Lys Leu Thr Pro Leu Val Leu Glu Val Phe Gly Asn
                   245                 250                 255
   Glu Ile Ser
<210> 7
   <211> 762
   <212> ADN
   <213> Séquence artificielle
<220>
   <223> Description de la séquence artificielle: séquence nucléotidique codant un polypeptide ndérivé du récepteur de la vitamine D humain
<220>
   <221> CDS
   <222> (1)..(759)
<400> 7
   ctg tct gag gag cag cag cgc atc att gcc ata ctg ctg gac gcc cac      48
   Leu Ser Glu Glu Gln Gln Arg Ile Ile Ala Ile Leu Leu Asp Ala His
   1               5                   10                  15
   cat aag acc tac gac ccc acc tac tcc gac ttc tgc cag ttc cgg cct      96
   His Lys Thr Tyr Asp Pro Thr Tyr Ser Asp Phe Cys Gln Phe Arg Pro
               20                  25                  30
   cca gtt cgt gtg aat gat ggt gga ggg agc gtg acc cta gag ctg tcc      144
   Pro Val Arg Val Asn Asp Gly Gly Gly Ser Val Thr Leu Glu Leu Ser
           35                  40                  45
   cag ctc tcc atg ctg ccc cac ctg gct gac ctg gtc agt tac agc atc      192
   Gln Leu Ser Met Leu Pro His Leu Ala Asp Leu Val Ser Tyr Ser Ile
       50                  55                  60
   caa aag gtc att ggc ttt gct aag atg ata cca gga ttc aga gac ctc      240
   Gln Lys Val Ile Gly Phe Ala Lys Met Ile Pro Gly Phe Arg Asp Leu
   65                  70                  75                  80
   acc tct gag gac cag atc gta ctg ctg aag tca agt gcc att gag gtc      288
   Thr Ser Glu Asp Gln Ile Val Leu Leu Lys Ser Ser Ala Ile Glu Val
                   85                  90                  95
   atc atg ttg cgc tcc aat gag tcc ttc acc atg gac gac atg tcc tgg    336
   Ile Met Leu Arg Ser Asn Glu Ser Phe Thr Met Asp Asp Met Ser Trp
               100                 105                 110
   acc tgt ggc aac caa gac tac aag tac cgc gtc agt gac gtg acc aaa    384
   Thr Cys Gly Asn Gln Asp Tyr Lys Tyr Arg Val Ser Asp Val Thr Lys
           115                 120                 125
   gcc gga cac agc ctg gag ctg att gag ccc ctc atc aag ttc cag gtg    432
   Ala Gly His Ser Leu Glu Leu Ile Glu Pro Leu Ile Lys Phe Gln Val
       130                 135                 140
   gga ctg aag aag ctg aac ttg cat gag gag gag cat gtc ctg ctc atg    480
   Gly Leu Lys Lys Leu Asn Leu His Glu Glu Glu His Val Leu Leu Met
   145                 150                 155                 160
   gcc atc tgc atc gtc tcc cca gat cgt cct ggg gtg cag gac gcc gcg    528
   Ala Ile Cys Ile Val Ser Pro Asp Arg Pro Gly Val Gln Asp Ala Ala
                   165                 170                 175
   ctg atc gag gcc atc cag gac cgc ctg tcc aac aca ctg cag acg tac    576
   Leu Ile Glu Ala Ile Gln Asp Arg Leu Ser Asn Thr Leu Gln Thr Tyr
               180                 185                 190
   atc cgc tgc cgc cac ccg ccc ccg ggc agc cac ctg ctc tat gcc aag    624
   Ile Arg Cys Arg His Pro Pro Pro Gly Ser His Leu Leu Tyr Ala Lys
           195                 200                 205
   atg atc cag aag cta gcc gac ctg cgc agc ctc aat gag gag cac tcc    672
   Met Ile Gln Lys Leu Ala Asp Leu Arg Ser Leu Asn Glu Glu His Ser
       210                 215                 220
   aag cag tac cgc tgc ctc tcc ttc cag cct gag tgc agc atg aag cta    720
   Lys Gln Tyr Arg Cys Leu Ser Phe Gln Pro Glu Cys Ser Met Lys Leu
   225                 230                 235                 240
   acg ccc ctt gtg ctc gaa gtg ttt ggc aat gag atc tcc tga            762
   Thr Pro Leu Val Leu Glu Val Phe Gly Asn Glu Ile Ser
                   245                 250
<210> 8
   <211> 253
   <212> PRT
   <213> Séquence artificielle
   <223> Description de la séquence artificielle: séquence nucléotidique codant un polypeptide ndérivé du récepteur de la vitamine D humain
<400> 8
   Leu Ser Glu Glu Gln Gln Arg Ile Ile Ala Ile Leu Leu Asp Ala His
    1               5                  10                  15
   His Lys Thr Tyr Asp Pro Thr Tyr Ser Asp Phe Cys Gln Phe Arg Pro
               20                  25                  30
   Pro Val Arg Val Asn Asp Gly Gly Gly Ser Val Thr Leu Glu Leu Ser
           35                  40                  45
   Gln Leu Ser Met Leu Pro His Leu Ala Asp Leu Val Ser Tyr Ser Ile
       50                  55                  60
   Gln Lys Val Ile Gly Phe Ala Lys Met Ile Pro Gly Phe Arg Asp Leu
   65                  70                  75                  80
   Thr Ser Glu Asp Gln Ile Val Leu Leu Lys Ser Ser Ala Ile Glu Val
                   85                  90                  95
   Ile Met Leu Arg Ser Asn Glu Ser Phe Thr Met Asp Asp Met Ser Trp
               100                 105                 110
   Thr Cys Gly Asn Gln Asp Tyr Lys Tyr Arg Val Ser Asp Val Thr Lys
           115                 120                 125
   Ala Gly His Ser Leu Glu Leu Ile Glu Pro Leu Ile Lys Phe Gln Val
       130                 135                 140
   Gly Leu Lys Lys Leu Asn Leu His Glu Glu Glu His Val Leu Leu Met
   145                 150                 155                 160
   Ala Ile Cys Ile Val Ser Pro Asp Arg Pro Gly Val Gln Asp Ala Ala
                   165                 170                 175
   Leu Ile Glu Ala Ile Gln Asp Arg Leu Ser Asn Thr Leu Gln Thr Tyr
               180                 185                 190
   Ile Arg Cys Arg His Pro Pro Pro Gly Ser His Leu Leu Tyr Ala Lys
           195                 200                 205
   Met Ile Gln Lys Leu Ala Asp Leu Arg Ser Leu Asn Glu Glu His Ser
       210                 215                 220
   Lys Gln Tyr Arg Cys Leu Ser Phe Gln Pro Glu Cys Ser Met Lys Leu
   225                 230                 235                 240
   Thr Pro Leu Val Leu Glu Val Phe Gly Asn Glu Ile Ser
                   245                 250